# EUROPEAN PATENT SPECIFICATION

(11) **EP 1 695 986 B1**
(45) Date of publication and mention of the grant of the patent: **14.12.2016**
(21) Application number: 06007998.5
(22) Date of filing: 09.10.1997
(51) Int. Cl.: C07K 16/32, A61K 39/395, C12N 15/13, C12N 5/20, G01N 33/577, G01N 33/68, A61P 35/00

(54) **Anti-erb B2 antibodies**
Antikörper gegen Erb B2
Anticorps contre erb B2

(30) Priority: 18.10.1996 US 731794
(43) Date of publication of application: 30.08.2006
(62) Divisional of application: 97912708.1
(73) Proprietor: Genentech, Inc., South San Francisco, CA 94080-4990 (US); BOARD OF REGENTS UNIVERSITY OF TEXAS SYSTEM, Austin, TX 78701-2981 (US)
(72) Inventor: Fendly, Brian M., South San Francisco, CA 94080-4990 (US); Phillips, Gail Dianne, San Carlos CA 94070 (US); Scheuermann, Richard H., Carrollton, TX 75007 (US); Uhr, Jonathan W., Dallas TX 75230 (US)
(74) Representative: Walton, Seán Malcolm

(56) References cited:
- Y. KITA ET AL.: "ErbB receptor activation, cell morphology changes, and apoptosis induced by anti-Her2 monoclonal antibodies.", BIOCHEMICAL AND BIOPHYSICAL RESEARCH COMMUNICATIONS, vol. 226, no. 1, 4 September 1996 (1996-09-04), pages 59-69, XP002027856, USA
- A. ILGEN ET AL.: "Characterization of anti-Her/2 antibodies which inhibit the growth of breast tumor cells in vitro.", PROCEEDINGS OF THE AMERICAN ASSOCIATION FOR CANCER RESEARCH, vol. 37, March 1996 (1996-03), page 470, XP002055529, USA
- A. ILGEN ET AL.: "Characterization of anti-Her/2 monoclonal antibodies which inhibit the growth of breast cancer cell lines in vitro.", PROCEEDINGS OF THE AMERICAN ASSOCIATION FOR CANCER RESEARCH, vol. 37, March 1996 (1996-03), page 84, XP002055530, USA
- B. FENDLY ET AL.: "Characterization of murine monoclonal antibodies reactive to either the human epidermal growth factor receptor or HER2/Neu gene product.", CANCER RESEARCH, vol. 50, no. 5, 1 March 1990 (1990-03-01), pages 1550-1558, XP002055531, USA
- C. MARTH ET AL.: "Effects of interferons on the expression of the proto-oncogene Her-2 in human ovarian carcinoma cells.", INTERNATIONAL JOURNAL OF CANCER, vol. 50, no. 1, 2 January 1992 (1992-01-02), pages 64-68, XP002055532,
- M. SLIWKOWSKI ET AL.: "A humanized monoclonal antibody for the treatment of HER2 overexpressing breast cancer.", PROCEEDINGS OF THE AMERICAN ASSOCIATION FOR CANCER RESEARCH, vol. 37, March 1996 (1996-03), pages 625-626, XP002055533, USA
- P. CARTER ET AL.: "Humanization of an anti-p185HER2 antibody for human cancer therapy.", PROCEEDINGS OF THE NATIONAL ACADEMY OF SCIENCES OF THE U.S.A., vol. 89, no. 10, 15 May 1992 (1992-05-15), pages 4285-4289, XP000275844, USA
- K. DE SANTES ET AL.: "Radiolabeled antibody targeting of the HER-2/Neu oncoprotein.", CANCER RESEARCH, vol. 52, no. 7, 1 April 1992 (1992-04-01), pages 1916-1923, XP002055534, USA

### SEQUENCE LISTING

(1) GENERAL INFORMATION:
   (i) APPLICANT: Genentech, Inc. Board of Regents, University of Texas System
   (ii) TITLE OF INVENTION: Anti-ErbB2 Antibodies
   (iii) NUMBER OF SEQUENCES: 9
   (iv) CORRESPONDENCE ADDRESS:
      (A) ADDRESSEE: Genentech, Inc.
      (B) STREET: 1 DNA Way
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Lee, Wendy M.
      (B) REGISTRATION NUMBER: 40,378
      (C) REFERENCE/DOCKET NUMBER: P1053R2PCT
   (ix) TELECOMMUNICATION INFORMATION:
      (A) TELEPHONE: 650/225-1994
      (B) TELEFAX: 650/952-9881
(2) INFORMATION FOR SEQ ID NO:1:
   (i) SEQUENCE CHARACTERISTICS:
      (A) LENGTH: 166 amino acids
      (B) TYPE: Amino Acid
      (D) TOPOLOGY: Linear
   (xi) SEQUENCE DESCRIPTION: SEQ ID NO:1:
   Cys Thr Gly Thr Asp Met Lys Leu Arg Leu Pro Ala Ser Pro Glu
     1               5                  10                  15
   Thr His Leu Asp Met Leu Arg His Leu Tyr Gln Gly Cys Gln Val
                    20                  25                  30
   Val Gln Gly Asn Leu Glu Leu Thr Tyr Leu Pro Thr Asn Ala Ser
                    35                  40                  45
   Leu Ser Phe Leu Gln Asp Ile Gln Glu Val Gln Gly Tyr Val Leu
                    50                  55                  60
   Ile Ala His Asn Gln Val Arg Gln Val Pro Leu Gln Arg Leu Arg
                    65                  70                  75
   Ile Val Arg Gly Thr Gln Leu Phe Glu Asp Asn Tyr Ala Leu Ala
                    80                  85                  90
   Val Leu Asp Asn Gly Asp Pro Leu Asn Asn Thr Thr Pro Val Thr
                    95                 100                 105
   Gly Ala Ser Pro Gly Gly Leu Arg Glu Leu Gln Leu Arg Ser Leu
                   110                 115                 120
   Thr Glu Ile Leu Lys Gly Gly Val Leu Ile Gln Arg Asn Pro Gln
                   125                 130                 135
   Leu Cys Tyr Gln Asp Thr Ile Leu Trp Lys Asp Ile Phe His Lys
                   140                 145                 150
   Asn Asn Gln Leu Ala Leu Thr Leu Ile Asp Thr Asn Arg Ser Arg
                   155                 160                 165
   Ala
   166
(2) INFORMATION FOR SEQ ID NO:2:
   (i) SEQUENCE CHARACTERISTICS:
      (A) LENGTH: 32 amino acids
      (B) TYPE: Amino Acid
      (D) TOPOLOGY: Linear
   (xi) SEQUENCE DESCRIPTION: SEQ ID NO:2:
   Ser Thr Gln Val Cys Thr Gly Thr Asp Met Lys Leu Arg Leu Pro
    1               5                  10                  15
   Ala Ser Pro Glu Thr His Leu Asp Met Leu Arg His Leu Tyr Gln
                   20                  25                  30
   Gly Cys
        32
(2) INFORMATION FOR SEQ ID NO:3:
   (i) SEQUENCE CHARACTERISTICS:
      (A) LENGTH: 59 amino acids
      (B) TYPE: Amino Acid
      (D) TOPOLOGY: Linear
   (xi) SEQUENCE DESCRIPTION: SEQ ID NO:3:
   Val Glu Glu Cys Arg Val Leu Gln Gly Leu Pro Arg Glu Tyr Val
    1               5                  10                  15
   Asn Ala Arg His Cys Leu Pro Cys His Pro Glu Cys Gln Pro Gln
                   20                  25                  30
   Asn Gly Ser Val Thr Cys Phe Gly Pro Glu Ala Asp Gln Cys Val
                   35                  40                  45
   Ala Cys Ala His Tyr Lys Asp Pro Pro Phe Cys Val Ala Arg
                  50                  55              59
(2) INFORMATION FOR SEQ ID NO:4:
   (i) SEQUENCE CHARACTERISTICS:
      (A) LENGTH: 97 amino acids
      (B) TYPE: Amino Acid
      (D) TOPOLOGY: Linear
   (xi) SEQUENCE DESCRIPTION: SEQ ID NO:4:
   Val Asn Cys Ser Gln Phe Leu Arg Gly Gln Glu Cys Val Glu Glu
    1               5                  10                  15
   Cys Arg Val Leu Gln Gly Leu Pro Arg Glu Tyr Val Asn Ala Arg
                   20                  25                  30
   His Cys Leu Pro Cys His Pro Glu Cys Gln Pro Gln Asn Gly Ser
                   35                  40                  45
   Val Thr Cys Phe Gly Pro Glu Ala Asp Gln Cys Val Ala Cys Ala
                   50                  55                  60
   His Tyr Lys Asp Pro Pro Phe Cys Val Ala Arg Cys Pro Ser Gly
                   65                  70                  75
   Val Lys Pro Asp Leu Ser Tyr Met Pro Ile Trp Lys Phe Pro Asp
                   80                  85                  90
   Glu Glu Gly Ala Cys Gln Pro
                   95      97
(2) INFORMATION FOR SEQ ID NO:5:
   (i) SEQUENCE CHARACTERISTICS:
      (A) LENGTH: 11 amino acids
      (B) TYPE: Amino Acid
      (D) TOPOLOGY: Linear
   (xi) SEQUENCE DESCRIPTION: SEQ ID NO:5:
   Pro Lys Asn Ser Ser Met Ile Ser Asn Thr Pro
    1               5                  10  11
(2) INFORMATION FOR SEQ ID NO:6:
   (i) SEQUENCE CHARACTERISTICS:
      (A) LENGTH: 7 amino acids
      (B) TYPE: Amino Acid
      (D) TOPOLOGY: Linear
   (xi) SEQUENCE DESCRIPTION: SEQ ID NO:6:
   His Gln Ser Leu Gly Thr Gln
    1               5       7
(2) INFORMATION FOR SEQ ID NO:7:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:7:
   His Gln Asn Leu Ser Asp Gly Lys
    1               5           8
(2) INFORMATION FOR SEQ ID NO:8:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:8:
   His Gln Asn Ile Ser Asp Gly Lys
    1               5           8
(2) INFORMATION FOR SEQ ID NO:9:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:9:
   Val Ile Ser Ser His Leu Gly Gln
    1               5           8

## Description

### BACKGROUND OF THE INVENTION

### Field of the Invention

This invention relates generally to antibodies which bind the ErbB2 receptor. In particular, it pertains to anti-ErbB2 antibodies which bind to an epitope in Domain 1 of ErbB2 and induce cell death via apoptosis.

### Description of Related Art

Transduction of signals that regulate cell growth and differentiation is modulated in part by phosphorylation of various cellular proteins. Protein tyrosine kinases are enzymes that are involved in this process. Receptor protein tyrosine kinases are believed to direct cellular growth via ligand-stimulated tyrosine phosphorylation of intracellular substrates. The class I subfamily of growth factor receptor protein tyrosine kinases includes the 170 kDa epidermal growth factor receptor (EGFR) encoded by the *erb*B1 gene. *erb*B1 has been causally implicated in human malignancy. In particular, increased expression of this gene has been observed in carcinomas of the breast, bladder, lung, head, neck and stomach. Monoclonal antibodies directed against the EGFR have been evaluated as therapeutic agents in the treatment of such malignancies. For a review, see Baselga et al. Pharmac. Ther. 64:127-154 (1994). See also Masui et al. Cancer Research 44:1002-1007 (1984).

Wu et al J. Clin. Invest. 95:1897-1905 (1995) recently report that the anti-EGFR monoclonal antibody (mAb) 225 (which competitively inhibits EGF binding and blocks activation of this receptor) could induce the human colorectal carcinoma cell line DiFi (which expresses high levels of EGFR) to undergo Gₗ cell cycle arrest and programmed cell death (apoptosis). Addition of IGF-1 or high concentrations of insulin could delay apoptosis induced by mAb 225, whereas Gₗ arrest could not be reversed by addition of IGF-1 or insulin.

The second member of the class I subfamily, p185*^{neu}*, was originally identified as the product of the transforming gene from neuroblastomas of chemically treated rats. The activated form of the *neu* protooncogene results from a point mutation (valine to glutamic acid) in the transmembrane region of the encoded protein. Amplification of the human homolog of *neu* (called *erbB2* or HER2) is observed in breast and ovarian cancers and correlates with a poor prognosis (Slamon et al., Science, 235:177-182 (1987); and Slamon et al., Science, 244:707-712 (1989)). Accordingly, Slamon et al. in US Pat No. 4,968,603 describe various diagnostic assays for determining *erb*B2 gene amplification or expression in tumor cells. To date, no point mutation analogous to that in the *neu* protooncogene has been reported for human tumors. Overexpression (frequently but not uniformly due to amplification) of *erb*B2 has also been observed in other carcinomas including carcinomas of the stomach, endometrium, salivary gland, lung, kidney, colon, thyroid, pancreas and bladder. See, among others, King et al., Science, 229:974 (1985); Yokota et al., Lancet: 1:765-767 (1986); Fukushigi et al., Mol Cell Biol., 6:955-958 (1986); Geurin et al., Oncogene Res., 3:21-31 (1988); Cohen et al., Oncogene, 4:81-88 (1989); Yonemura et al., Cancer Res., 51:1034 (1991); Borst et al., Gynecol. Oncol, 38:364 (1990); Weiner et al., Cancer Res., 50:421-425 (1990); Kern et al., Cancer Res., 50:5184 (1990); Park et al, Cancer Res., 49:6605 (1989); Zhau et al., Mol. Carcinog., 3:354-357 (1990); Aasland et al. Br. J. Cancer 57:358-363 (1988); Williams et al. Pathiobiology 59:46-52 (1991); and McCann et a/., Cancer, 65:88-92 (1990).

Antibodies directed against the rat *neu* and human *erb*B2 protein products have been described. Drebin et al., Cell 41:695-706 (1985) refer to an IgG2a monoclonal antibody which is directed against the rat *neu* gene product. This antibody called 7.16.4 causes down-modulation of cell surface p185 expression on B104-1-1 cells (NIH-3T3 cells transfected with the *neu* protooncogene) and inhibits colony formation of these cells. Drebin *et al.* say at page 699 that the antibody exerts a cytostatic effect rather than an irreversible cytotoxic effect on *neu*-transformed cells in soft agar. In Drebin et al. PNAS (USA) 83:9129-9133 (1986), the 7.16.4 antibody was shown to inhibit the tumorigenic growth of *neu*-transformed NIH-3T3 cells as well as rat neuroblastoma cells (from which the *neu* oncogene was initially isolated) implanted into nude mice. Drebin et al. in Oncogene 2:387-394 (1988) discuss the production of a panel of antibodies against the rat *neu* gene product. All of the antibodies were found to exert a cytostatic effect on the growth of *neu*-transformed cells suspended in soft agar. Antibodies of the IgM, IgG2a and IgG2b isotypes were able to mediate significant *in vitro* lysis of *neu*-transformed cells in the presence of complement, whereas none of the antibodies were able to mediate high levels of antibody-dependent cellular cytotoxicity (ADCC) of the *neu*-transfotmed cells. Drebin et al. Oncogene 2:273-277 (1988) report that mixtures of antibodies reactive with two distinct regions on the p185 molecule result in synergistic anti-tumor effects on *neu*-transformed NIH-3T3 cells implanted into nude mice. Biological effects of anti-*neu* antibodies are reviewed in Myers et al., Melh. Enzym. 198:277-290 (1991). See also WO94/22478 published October 13, 1994.

Hudziak et al., Mol. Cell Biol. 9(3):1165-1172 (1989) describe the generation of a panel of anti-ErbB2 antibodies which were characterized using the human breast tumor cell line SKBR3. Relative cell proliferation of the SKBR3 cells following exposure to the antibodies was determined by crystal violet staining of the monolayers after 72 hours. Using this assay, maximum inhibition was obtained with the antibody called 4D5 which inhibited cellular proliferation by 56%. Other antibodies in the panel, including 7C2 and 7F3, reduced cellular proliferation to a lesser extent in this assay. Hudziak *et al.* conclude that the effect of the 4D5 antibody on SKBR3 cells was cytostatic rather than cytotoxic, since SKBR3 cells resumed growth at a nearly normal rate following removal of the antibody from the medium. The antibody 4D5 was further found to sensitize p185^{*erb*B2}-overexpressing breast tumor cell lines to the cytotoxic effects of TNF-α. See also WO89/06692 published July 27, 1989. The anti-ErbB2 antibodies discussed in Hudziak *et al* are further characterized in Fendly et al. Cancer Research 50:1550-1558 (1990); Kotts et al. In Vitro 26(3):59A (1990); Sarup et al. Growth Regulation 1:72-82 (1991); Shepard et al. J. Clin. Immunol. 11(3):117-127 (1991); Kumar et al. Mol. Cell. Biol. 11(2):979-986 (1991); Lewis et al. Cancer Immunol. Immunother. 37:255-263 (1993); Pietras et al. Oncogene 9:1829-1838 (1994); Vitetta et al. Cancer Research 54:5301-5309 (1994); Sliwkowski et al. J. Biol. Chem. 269(20):14661-14665 (1994); Scott et al. J. Biol. Chem. 266:14300-5 (1991); and D'souza et al. Proc. Natl. Acad. Sci. 91:7202-7206 (1994).

Tagliabue et al. Int. J Cancer 47:933-937 (1991) describe two antibodies which were selected for their reactivity on the lung adenocarcinoma cell line (Calu-3) which overexpresses ErbB2. One of the antibodies, called MGR3. was found to internalize, induce phosphorylation of ErbB2, and inhibit tumor cell growth *in vitro.*

McKenzie et al. Oncogene 4:543-548 (1989) generated a panel of anti-ErbB2 antibodies with varying epitope specificities, including the antibody designated TA1. This TA1 antibody was found to induce accelerated endocytosis of ErbB2 (see Maier et al. Cancer Res. 51:5361-5369 (1991)). Bacus et al. Molecular Carcinogenesis 3:350-362 (1990) reported that the TA1 antibody induced maturation of the breast cancer cell lines AU-565 (which overexpresses the *erb*B2 gene) and MCF-7 (which does not). Inhibition of growth and acquisition of a mature phenotype in these cells was found to be associated with reduced levels of ErbB2 receptor at the cell surface and transient increased levels in the cytoplasm.

Stancovski et al. PNAS (USA) 88:8691-8695 (1991) generated a panel of anti-ErbB2 antibodies, injected them i.p. into nude mice and evaluated their effect on tumor growth of murine fibroblasts transformed by overexpression of the *erb*B2 gene. Various levels of tumor inhibition were detected for four of the antibodies, but one of the antibodies (N28) consistently stimulated tumor growth. Monoclonal antibody N28 induced significant phosphorylation of the ErbB2 receptor, whereas the other four antibodies generally displayed low or no phosphorylation-inducing activity. The effect of the anti-ErbB2 antibodies on proliferation of SKBR3 cells was also assessed. In this SKBR3 cell proliferation assay, two of the antibodies (N12 and N29) caused a reduction in cell proliferation relative to control. The ability of the various antibodies to induce cell lysis *in vitro* via complement-dependent cytotoxicity (CDC) and antibody-mediated cell-dependent cytotoxicity (ADCC) was assessed, with the authors of this paper concluding that the inhibitory function of the antibodies was not attributed significantly to CDC or ADCC.

Bacus et al. Cancer Research 52:2580-2589 (1992) further characterized the antibodies described in Bacus *et al*. (1990) and Stancovski *et al.* of the preceding paragraphs. Extending the i.p. studies of Stancovski *et al.,* the effect of the antibodies after i.v. injection into nude mice harboring mouse fibroblasts overexpressing human ErbB2 was assessed. As observed in their earlier work, N28 accelerated tumor growth whereas N12 and N29 significantly inhibited growth of the ErbB2-expressing cells. Partial tumor inhibition was also observed with the N24 antibody. Bacus *et al*. also tested the ability of the antibodies to promote a mature phenotype in the human breast cancer cell lines AU-565 and MDA-MB453 (which overexpress ErbB2) as well as MCF-7 (containing low levels of the receptor). Bacus *et al.* saw a correlation between tumor inhibition *in vivo* and cellular differentiation: the tumor-stimulatory antibody N28 had no effect on differentiation, and the tumor inhibitory action of the N12, N29 and N24 antibodies correlated with the extent of differentiation they induced.

Xu et al. Int. J Cancer 53:401-408 (1993) evaluated a panel of anti-ErbB2 antibodies for their epitope binding specificities, as well as their ability to inhibit anchorage-independent and anchorage-dependent growth of SKBR3 cells (by individual antibodies and in combinations), modulate cell-surface ErbB2. and inhibit ligand stimulated anchorage-independent growth. See also WO94/00136 published Jan 6, 1994 and Kasprzyk et al. Cancer Research 52:2771-2776 (1992) concerning anti-ErbB2 antibody combinations. Other anti-ErbB2 antibodies are discussed in Hancock et al. Cancer Res. 51:4575-4580 (1991); Shawver et al. Cancer Res. 54:1367-1373 (1994); Arteaga et al. Cancer Res. 54:3758-3765 (1994); and Harwerth et al. J. Biol. Chem. 267:15160-15167 (1992).

A further gene related to *erbB2,* called *erb*B3 or HER3, has also been described. See, *e.g.,* US Pat. Nos. 5,183,884 and 5,480,968. ErbB3 is unique among the ErbB receptor family in that it possesses little or no intrinsic tyrosine kinase activity. However, when ErbB3 is co-expressed with ErbB2, an active signaling complex is formed and antibodies directed against ErbB2 are capable of disrupting this complex (Sliwkowski et al., J. Biol Chem., 269(20):14661-14665 (1994)). Additionally, the affinity of ErbB3 for heregulin (HRG) is increased to a higher affinity state when co-expressed with ErbB2. See also, Levi et al., Journal of Neuroscience 15: 1329-1340(1995); Morrissey et al., Proc. Natl. Acad. Sci. USA 92:1431-1435 (1995); and Lewis et al., Cancer Res., 56:1457-1465 (1996) with respect to the ErbB2-ErbB3 protein complex.

The class I subfamily of growth factor receptor protein tyrosine kinases has been further extended to include the HER4/p180^{*erb*B4} receptor. See EP Pat Appln No 599,274; Plowman et al., Proc. Natl. Acad. Sci. USA, 90:1746-1750 (1993); and Plowman et al., Nature, 366:473-475 (1993). Plowman *et al.* found that increased HER4 expression correlated with certain carcinomas of epithelial origin, including breast adenocarcinamas. This receptor, like ErbB3. forms an active signalling complex with ErbB2 (Carraway and Cantley, Cell 78:5-8 (1994)).

The quest for an ErbB2 activator has lead to the discovery of a family of heregulin polypeptides. These proteins appear to result from alternative splicing of a single gene and are called neuregulins (NRGs), neu differentiation factors (NDFs), heregulins (HRGs), glial growth factors (GGFs) and acetylcholine receptor inducing activity (ARIA) in the literature. For a review, see Groenen et al. Growth Factors 11:235-257 (1994): Lemke. G. Molec. & Cell. Neurosci. 7:247-262 (1996) and Lee et al. Pharm. Rev. 47:51-85 (1995).

KITA et al. (1996) BIOCHEM. BIOPHYS. RES. COMM. 226(1), 59-69 discloses mAb74 binds ErbB2 and induces apoptosis in tumor cells.

PROC. AM. ASS. CANC. RES. (1996) vol. 37, p. 470, abstract #3209, p. 84, abstract #564, both ILGEN et al., disclose antibodies that bind the extracellular domain of ErbB2 and can inhibit growth of cancer cell lines via cell cycle arrest and apoptosis.

MARTH et al. (1992) INT. J. CANC. 50(1), 64-68 discloses that mAb 7C2 binds to the extracellular domain of ErbB2 and inhibits cancer cell growth.

SLIWKOWSKI et al. PROC. AM. ASS. CANC. RES. (1996) vol. 37, 625-626, and DE SANTES et al. (1992) CANC. RES. 52(7),1916-1923 disclose a humanized anti-ErbB2 antibody 4D5 in clinical trials, acting via cell growth arrest and ADCC.

### SUMMARY OF THE INVENTION

The present invention provides the subject-matter of claim 1, a composition for use in a method of therapeutic treatment in a mammal of a cancer characterized by overexpression of ErbB2, the method comprising administering to the mammal the composition comprising an effective amount of a first anti-ErbB2 antibody, which antibody binds to epitope 7C2/7F3, as defined in SEQ ID NO: 2, on Domain 1 of ErbB2 and induces cell death via apoptosis of a cancer cell of the mammal which overexpresses ErbB2 when the cancer cell is exposed to the antibody, the method further comprising administering to the mammal a second anti-ErbB2 antibody, which second antibody binds to epitope 4D5, as defined in SEQ ID NO: 4, on ErbB2 and inhibits growth of SKBR3 cells in cell culture by 50-100%, wherein the cancer cell of the mammal is exposed to the first antibody before it is exposed to the second antibody, by the method comprising sequential administration of the first antibody followed by the second antibody.

Preferred antibodies are monoclonal antibodies e.g. humanized antibodies. Antibodies of particular interest are those which, in addition to the above-described properties, bind the ErbB2 receptor with an affinity of at least about 10 nM, more preferably at least about 1 nM.

In Example 2 below, it was found that the pro-apoptotic antibody 7C2 almost completely eradicated the entire culture of growth arrested cells. Therefore, it may be desirable to combine the pyro-apoptotic antibodies disclosed herein with a growth inhibitory agent in the *in vitro* and *in vivo* methods discussed above. In such embodiments, superior levels of apoptosis may be achieved by administering the growth inhibitory agent prior to the pro-apoptotic anti-ErbB2 antibody. However, simultaneous administration or administration of the anti-ErbB2 antibody first is also contemplated.

### Brief Description of the Drawings

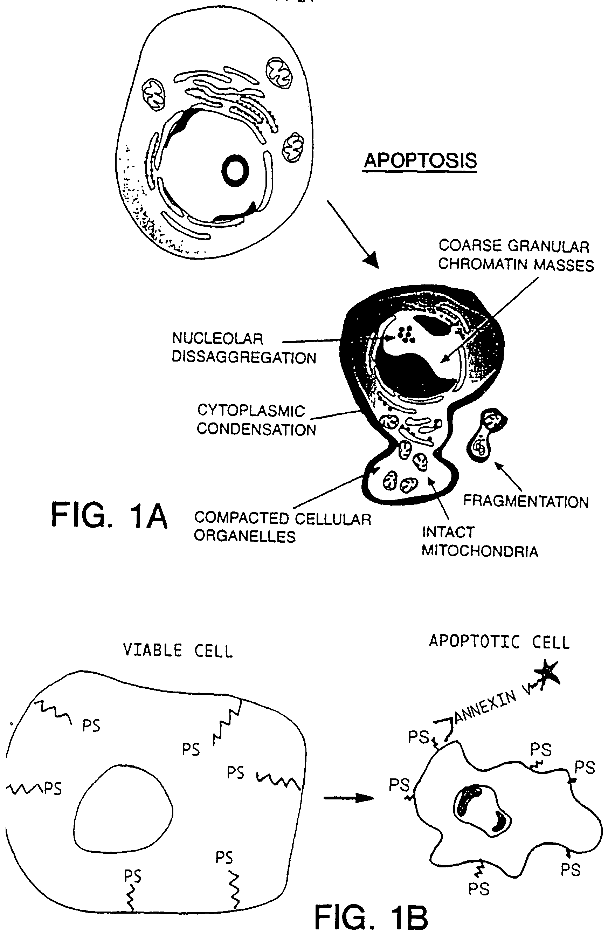

Figs. 1A & B show the effects of apoptosis on a cell and a method for determining apoptosis, respectively. Fig. 1A shows the physiological changes which occur to a cell which undergoes programmed cell death (apoptosis). Fig. 1B shows translocation of phosphatidyl serine (PS) from the inner leaflet of the plasma membrane to the exterior of the cell. This PS translocation process is specific for cells undergoing apoptosis. Annexin V binds specifically to PS and thus provides a means for determining apoptosis.

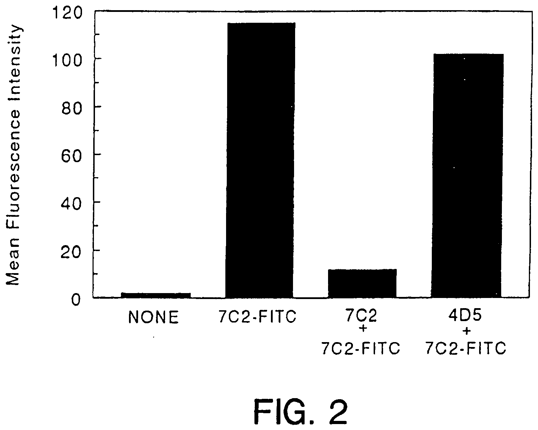

Fig. 2 shows the epitope specificity of anti-ErbB2 antibodies. MAbs were used to block the binding of 7C2 to BT474 cells. BT474 cells (0.5 x 10⁶) pretreated or not with 50µg anti-ErbB2 antibodies (15 minutes on ice) were washed twice, resuspended in 0.1ml 1% FBS/PBS and incubated with 5µg of fluoresceinisothioc-yanate (FITC) conjugated 7C2 antibody (15 minutes on ice). Following incubation, the cell suspensions were washed twice with 1% FBS/PBS to remove unbound fluorochrome, fixed with 1% paraformaldehyde/PBS and analyzed by flow cytometry.

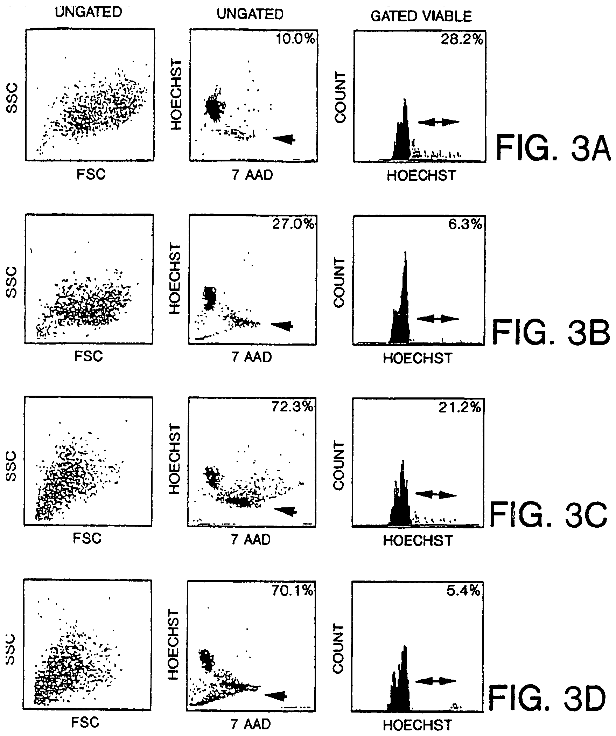

Figs. 3A-D depict the effect of anti-ErbB2 antibodies on human breast cancer cells which overexpress ErbB2. Cells with normal membrane functions at the time of harvest ("viable" cells) exclude the DNA dye 7AAD and after membrane permeabilization are stained preferentially with Hoechst. Analysis of these cells reveals a classic DNA profile (far right panels) with cells in the G₀/G₁ phase (major peak) and S-G₂-M phase (indicated with double headed arrow) of the cell cycle. In the center panels, the population of cells indicated by the arrow represent dead cells, some of which had abnormal permeability characteristics at the time of harvest and had degraded their nuclear DNA. 4 x 10⁴ BT474 cells/ml were incubated for 72 hours in medium containing an isotype-matched control 1g (Fig. 3A), 1µg of monoclonal 4D5 (Fig. 3B), 50µg or monoclonal 7C2 (Fig. 3C), or 1µg 4D5 antibody + 50µg 7C2 antibody (Fig. 3D). The percentage of dead cells is indicated in the middle panels (7AAD fluorescence vs. Hoechst fluorescence) and the percentage of viable cells in the combined S, G₂, and M phases of the cell cycle is indicated in the right panels (Hoechst fluorescence vs. cell count). The lefthand panel shows the size of the cells as determined by forward and right angle light scatter.

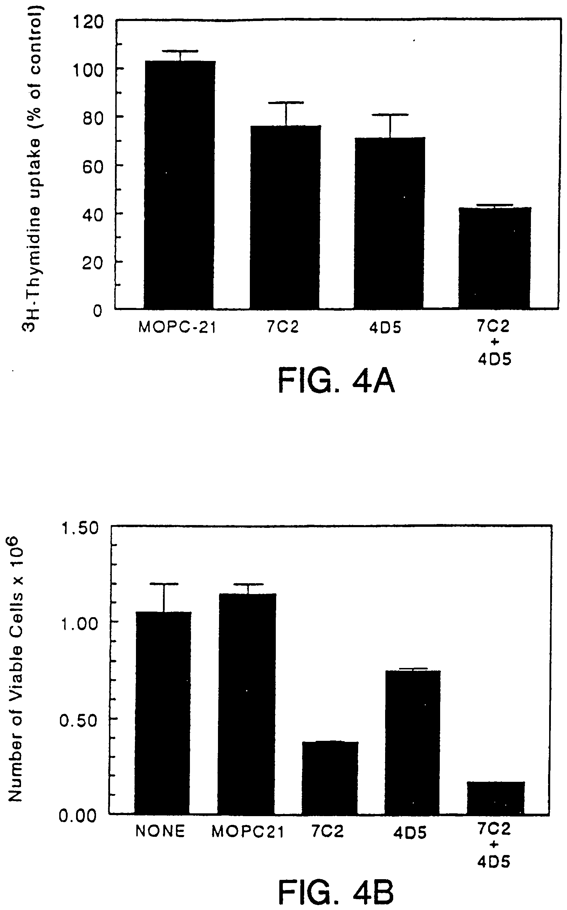

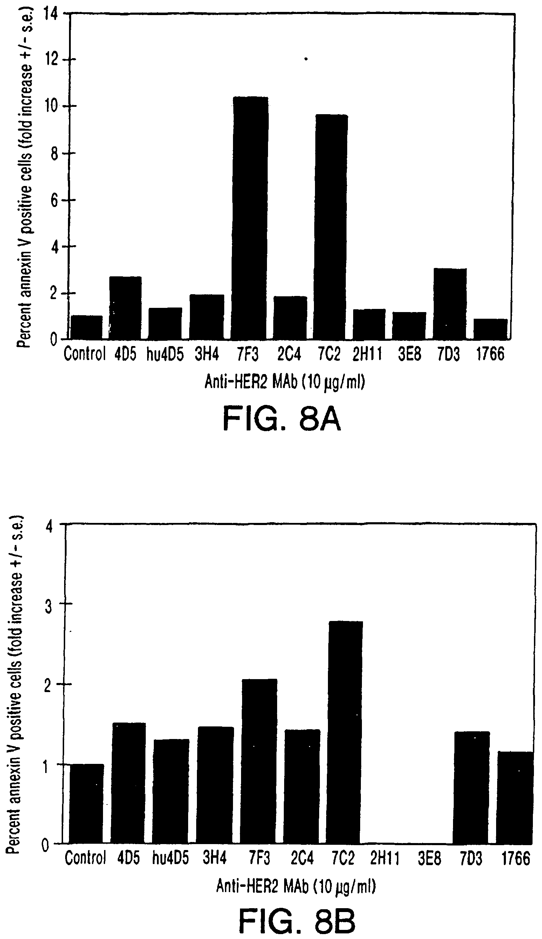

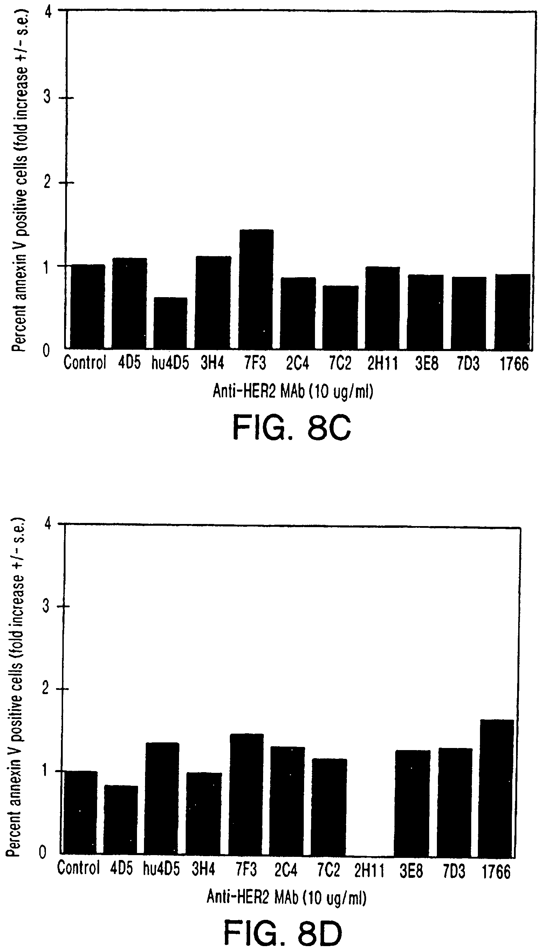

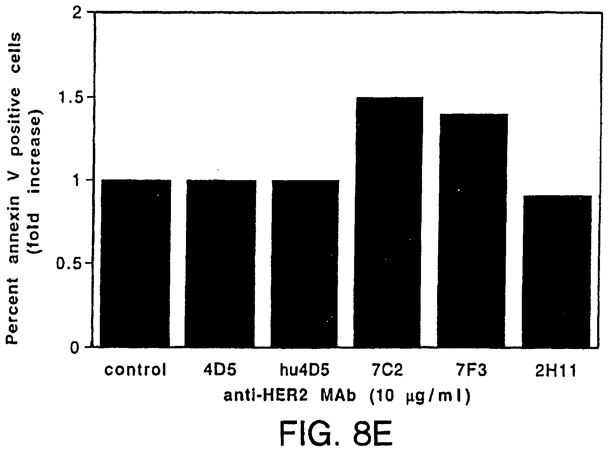

Figs. 4A & B reveal the additive effect of 4D5 and 7C2 antibodies on DNA synthesis, and viability in BT474 cells. The average of two experiments ± S.D. is shown. In Fig. 4A, 8 x 10³ cells/0.2 ml/well were treated with 4D5 (0.05µg/ml) and/or 7C2 (50µg/ml) for 72 hours and pulsed for the last 12 hours with 1µCi [³H]-thymidine (in triplicate). In Fig. 4B, for cell viability, a total cell count was obtained and viability was determined by FACS analysis. The standard deviation for both 7C2 and 7C2 plus 4D5 treatment is too small. (1 x 10³ cells) to be seen in the figure.

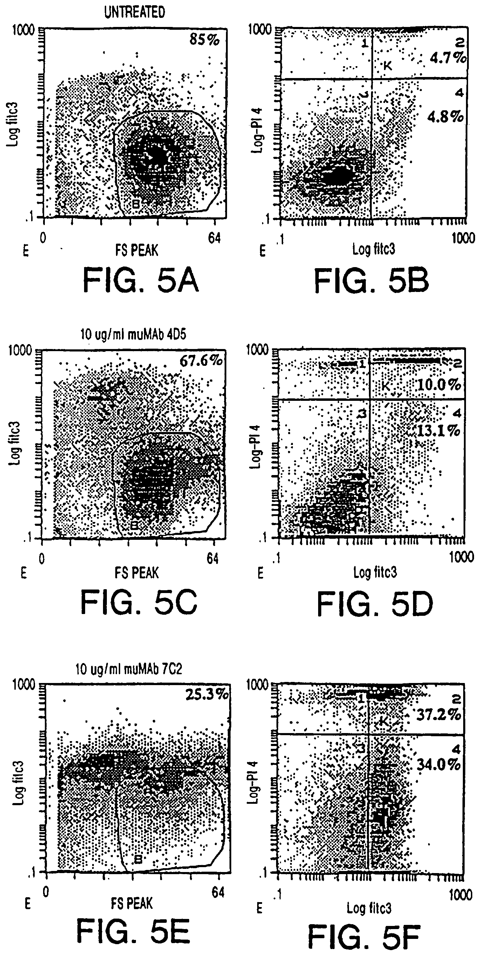

Figs. 5A-F show induction of apoptosis by anti-ErbB2 MAbs 7C2 and 4D5 in BT474 breast tumor cells. Figs. 5A, 5C and 5E show plots of forward scatter (FS), an indicator of cell size, vs. log FITC (representing annexin V binding). Figs. 5B, D and F are quadrant plots of log FITC vs. log propidium iodine (PI), with percent annexin V-positive cells shown in quadrant 4 and percent annexin V/PI positive cells in quadrant 2. Untreated cells display a uniform size and fluorescence signal, as seen within the drawn circle, and are 85% viable (Fig. 5A). In addition to displaying low annexin V-binding, these cells do not take up PI. indicating no change in membrane integrity. Treatment for 3 days with 10µg/ml MAb 7C2 results in a reduction in the percent of viable cells (to 25.3 %, Fig. 5E) and a shift of almost the entire population to a smaller, FITC-positive population of cells (Fig. 5E). As shown in Fig. 5F, MAb 7C2 induces a 7-8 fold increase in the percent of annexin V-positive/PI-positive cells, indicating apoptotic cell death. The anti-proliferative MAb, 4D5, induces a small degree of apoptosis (2.5 fold above control, Figs. 5C and D).

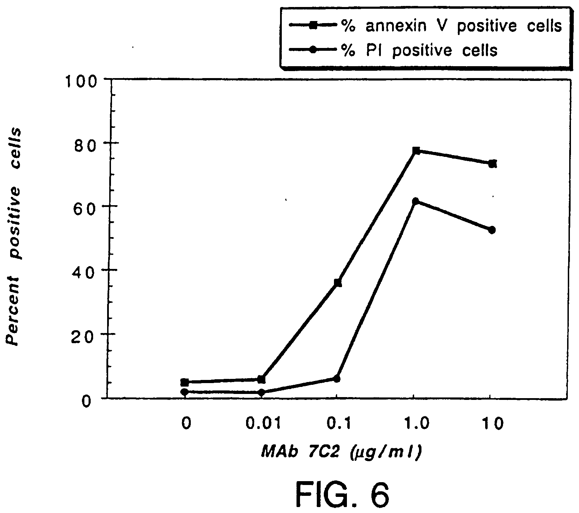

Fig. 6 shows that the effects of MAb 7C2 are dose-dependent. The induction of apoptosis in BT474 breast tumor cells by MAb 7C2. as measured by an increase in the number of annexin V-positive and PI-positive cells, is apparent at a concentration of 0.1µg/ml and reaches a maximum at 1µg/ml.

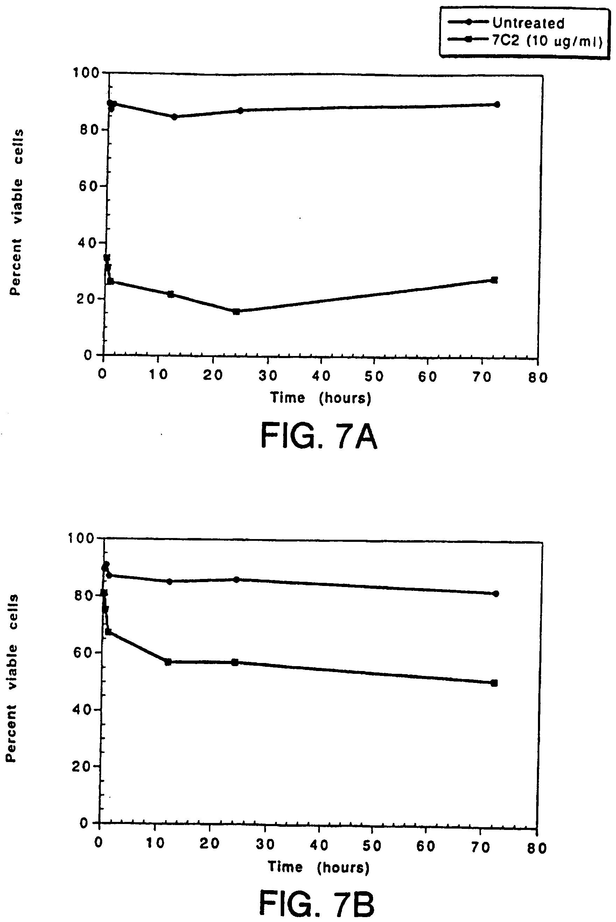

Figs. 7A and 7B are time-courses of MAb 7C2-induced apoptosis in BT474 and SKBR3 breast tumor cells, respectively. Treatment of BT474 cells (Fig. 7A) and SKBR3 cells (Fig 7B) with 10µg/ml MAb 7C2 results in a reduction in the percent of viable cells (those cells which are annexin V- and PI-negative) as early as 15 minutes after initiation of treatment and reaches a maximum at 24 hours. The BT474 cell line is more sensitive to the pro-apoptotic effect of MAb 7C2 compared to the SKBR3 cells.
Figs 8A-E show responses of different cell lines to anti-ErbB2 MAbs. The BT474, SKBR3 and MCF7 breast tumor cell lines, and normal human mammary epithelial cells (HMEC) (Figs. 8A-D, respectively) were incubated with the anti-ErbB2 MAbs 4D5, 3H4, 7F3, 7C2, 2H11, 3E8, and 7D3; the humanized version of muMAb 4D5 (hu4D5); or the isotype-matched irrelevant control MAb 1766. Treatment was for 3 days at a MAb concentration of 10µg/ml. The data are pooled from 2-9 separate experiments and are represented as mean fold increase (+/-s.e.) in annexin V binding over control cells. The response of the BT474 breast tumor cells to MAbs 7C2 and 4D5 is as described for Fig. 5 (9-fold and 2.5-fold above control, respectively). Induction of apoptosis in the SKBR3 breast tumor cell line, which expresses high levels of ErbB2 similar to the BT474 cells, also occurs after treatment with MAb 7C2 (and to a smaller degree, MAb 4D5). In addition. MAb 7F3 induces apoptosis in both the BT474 and SKBR3 cell lines. The MCF7 breast tumor line, which expresses normal ErbB2 levels, and the HMEC's showed no change in annexin V binding after treatment with the anti-ErbB2 Mabs. These results suggest that overexpression of ErbB2 is required for responsiveness to the anti-ErbB2 MAbs. In Fig. 8E, a non-small cell lung carcinoma line overexpressing ErbB2, Calu 3, was tested for induction of apoptosis by anti-ErbB2 MAbs. Treatment with 7C2 or 7F3 resulted in enhanced binding of annexin V.

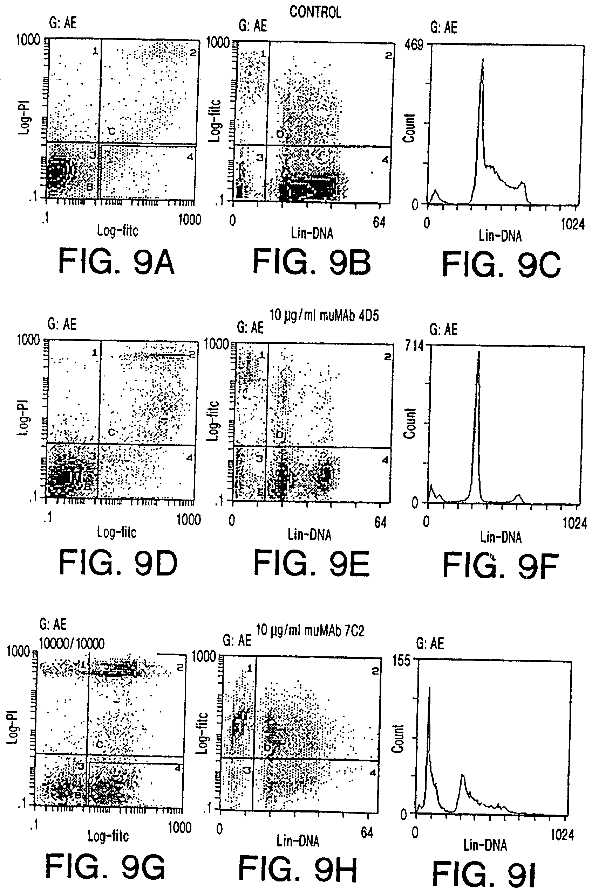

Figs. 9A-1 show the effects of MAbs 7C2 and 4D5 on cell cycle progression and cell death. Untreated BT474 cells are largely annexin V-negative and PI-negative (Fig. 9A, quadrant 3), and show a normal cell cycle DNA histogram (Figs. 9B &C). Cells treated with 10µg/ml MAb 4D5 show some increase in uptake of P1 and annexin V-FITC binding (Fig. 9D, quadrant 2). The most pronounced effect is on cell cycle progression, where MAb 4D5 almost completely reduces the percent of cells in S phase (Figs. 9E &F). MAb 7C2 induces a significant amount of cell death in BT474, as measured by PI uptake and annexin V-FITC binding (Fig. 9G, quadrant 2). Cell cycle analysis shows the presence of a sub-G₀/G₁ or hypodiploid population (Fig. 9I), characteristic of apoptotic cells, with the cells displaying high levels of annexin V binding (Fig. 9H, quadrant 1).

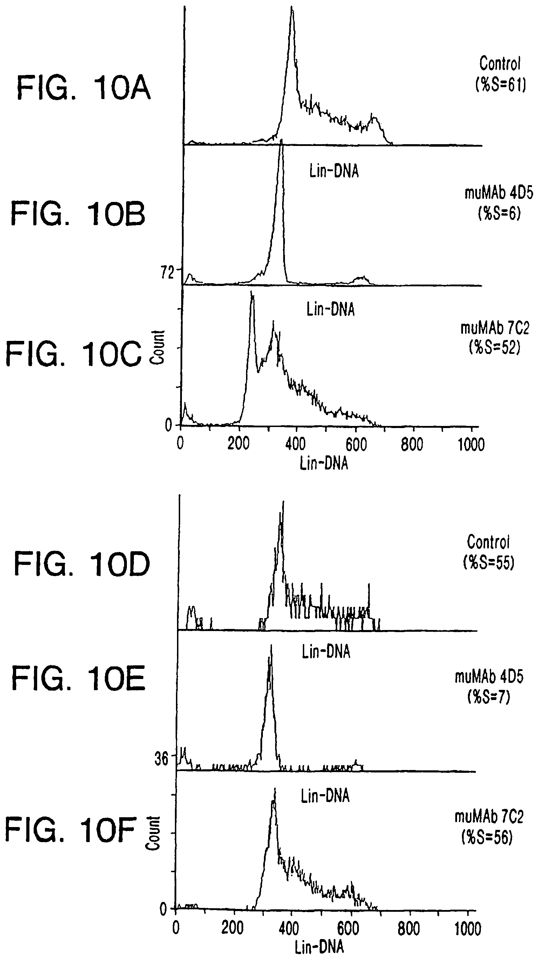

Figs. 10A-F are the results from curve-fitting analyses of the DNA histograms of Figs. 9A-I and show little change in the percent of cells in S-phase (52%) after MAb 7C2 treatment compared to control cells (61% S-phase cells), but a dramatic reduction in the number of cells in S-phase (to 6%) in response to MAb 4D5 (Figs. 10C, A and B, respectively). Analyses of the apoptotic population of cells (annexin V/PI positive cells from quadrant 2, 9A, D and G) reveal no difference in the percent S-phase cells compared to the total cell population (Fig. 10D control=55%; Fig. 10E MAb 4D5=7%; Fig. 10F MAb 7C2=56%). Furthermore, the G₀/G₁ and G₂/M phases show no change (compare Figs. 10A and D, B and E, C and F), indicating that cells are exiting the cell cycle at all phases and that MAb-induced apoptotic cell death is not cell cycle specific.

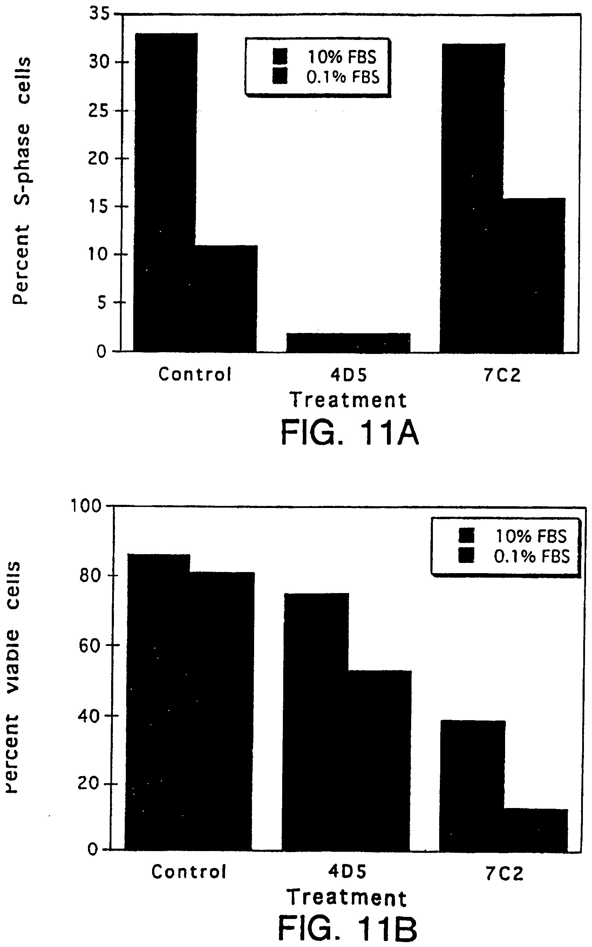

Figs. 11A & B show MAb 7C2-induced apoptosis is enhanced by growth arrest. In addition to the data from cell cycle studies, it was observed, from video time-lapse recording, that a proportion of MAb 7C2-treated cells continue to proliferate while others undergo apoptosis. Therefore, experiments were performed to determine if inhibition of cell growth would enhance the pro-apoptotic activity of MAb 7C2. BT474 cells were serum-deprived for 3 days to induce growth arrest, then treated with 10µg/ml MAb 7C2 or 4D5 for 3 days and analyzed for viability (annexin V-FITC binding and PI uptake) as well as cell cycle effects. Serum-deprivation (by incubation in media supplemented with 0.1% FBS) effectively reduces proliferation, as seen by a decrease in the percent of S-phase cells from 33% (in 10% FBS) to 10% (Fig. 11A). The potent anti-proliferative activity of MAb 4D5 is not further enhanced by prior growth arrest. The proportion of cells in S-phase, with and without serum-deprivation, in MAb 7C2-treated cells was similar to controls. Fig. 11B shows that serum-starvation does not adversely affect cell viability of untreated cells. However, viability is reduced to 55% in BT474 cells treated with 10µg/ml MAb 4D5 after a period of serum-deprivation. Moreover, treatment of growth-arrested cells with 10µg/ml MAb 7C2 almost completely eradicates the entire culture, in that the percent of annexin V-negative and PI-negative cells is reduced to 10%.

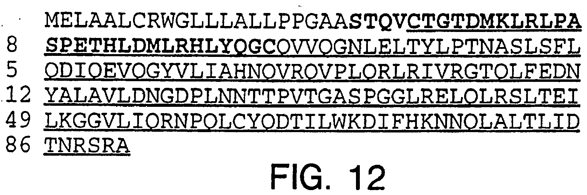

Fig. 12 depicts with underlining the amino acid sequence of Domain 1 of ErbB2 (SEQ ID NO:1). Bold amino acids indicate the location of the epitope recognized by MAbs 7C2 and 7F3 as determined by deletion mapping, *i.e*. the "7C2/7F3 epitope" (SEQ ID NO:2).

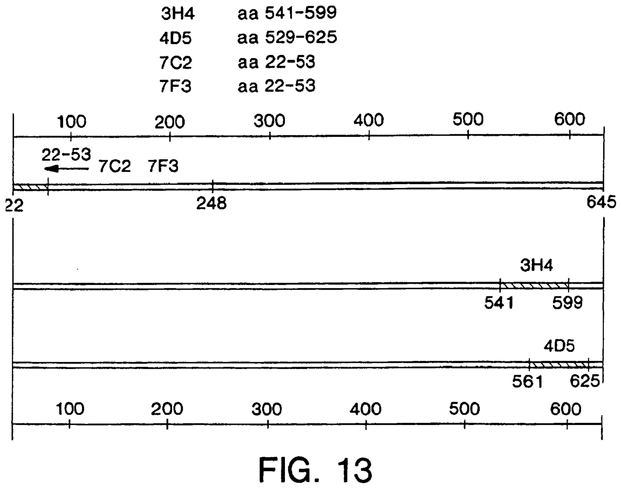

Fig. 13 shows epitope-mapping of the extracellular domain of ErbB2 as determined by truncation mutant analysis and site-directed mutagenesis (Nakamura et al. J. of Virology 67(10):6179-6191 (Oct 1993); Renz et al J. Cell Biol. 125(6):1395-1406 (Jun 1994)). Pro-apoptotic MAbs 7C2 and 7F3 bind an epitope at the N-terminus of the receptor, whereas anti-proliferative MAbs 4D5 and 3H4 bind adjacent to the transmembrane domain. The various ErbB2-ECD truncations or point mutations were prepared from cDNA using polymerase chain reaction technology. The ErbB2 mutants were expressed as gD fusion proteins in a mammalian expression plasmid. This expression plasmid uses the cytomegalovirus promoter/enhancer with SV40 termination and polyadenylation signals located downstream of the inserted cDNA. Plasmid DNA was transfected into 293S cells. One day following transfection, the cells were metabolically labeled overnight in methionine and cysteine-free, low glucose DMEM containing 1% dialyzed fetal bovine serum and 25 µCi each of ³⁵S methionine and³⁵ S cysteine. Supernatants were harvested either the ErbB2 MAbs or control antibodies were added to the supernatant and incubated 2-4 hours at 4°C. The complexes were precipitated, applied to a 10-20% Tricine SDS gradient gel and electrophoresed at 100 V. The gel was electroblotted onto a membrane and analyzed by autoradiography.

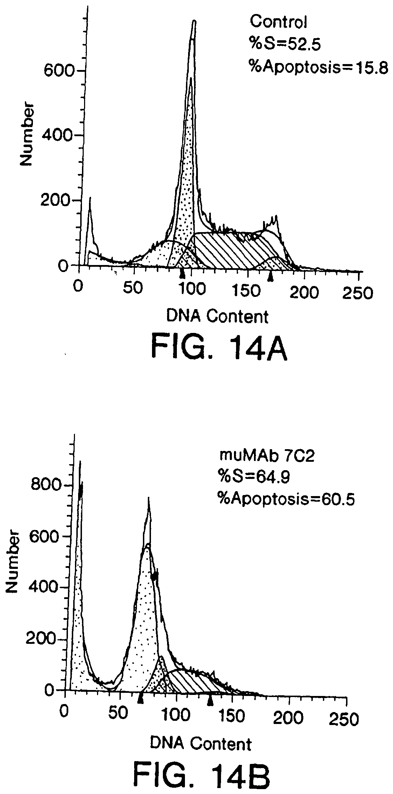

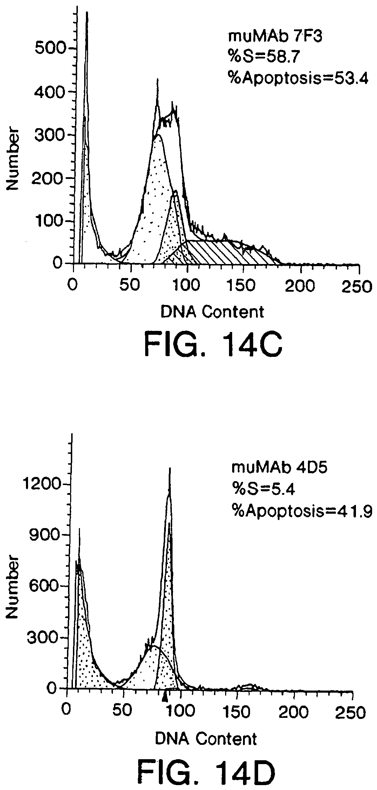

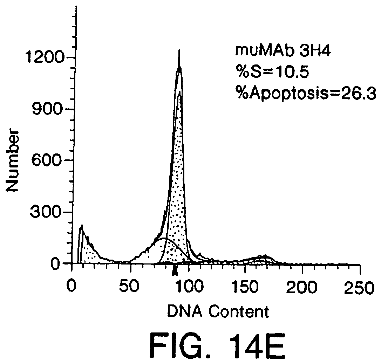

Figs. 14A-E show that the effects of anti-ErbB2 MAbs are epitope-specific. To determine if the anti-proliferative or pro-apoptotic effects of anti-ErbB2 MAbs are related to epitope specificity, BT474 cells were treated with 4 different MAbs for 3 days and stained with Hoechst 33342 for cell cycle analysis. The MAbs were: 7C2 and 7F3, which bind amino acids 22-53 (SEQ ID NO:2) on the ErbB2 extracellular domain (Figs.. 14B and C, respectively); 4D5, which binds residues 529-625 (SEQ ID NO:4) (Fig. 14D); and 3H4, which binds amino acids 541-599 (SEQ ID NO:3) (Fig. 14E). Both 7C2 and 7F3 induce apoptosis (to 60.5% and 53.4%, respectively, of the cell population), but did not decrease the proportion of S-phase cells (64.9% and 58.7%, respectively) compared to untreated cells (52.5%; Fig. 14A). In contrast, MAbs 4D5 and 3H4, which bind adjacent to the ErbB2 transmembrane region, show potent anti-proliferative activity (%S=5.4 and 10.5, respectively, control S=52.5%), but are not as effective as 7C2 or 7F3 in promoting apoptotic cell death (% apoptosis for 4D5=41.9, for 3H4=26.3, controls=15.8%).

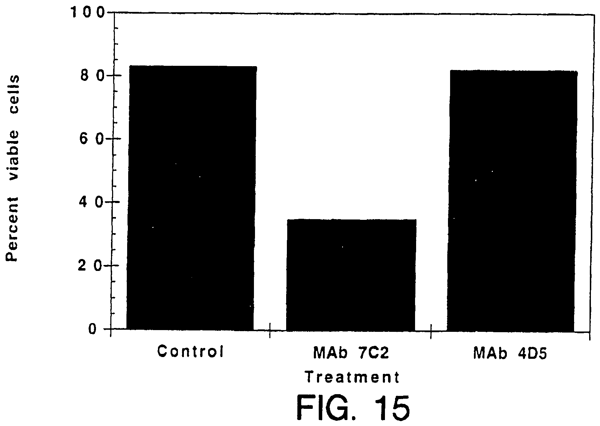

Fig. 15 shows induction of apoptosis by anti-HER2 MAb 7C2 in the SKOV3 ovarian carcinoma cell line as determined in Example 3.

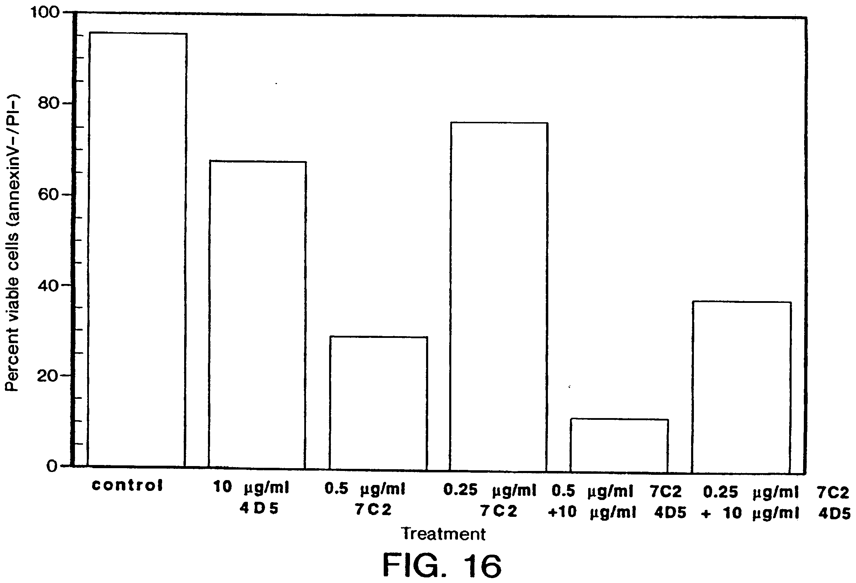

Fig. 16 shows that combination treatment with anti-HER2 MAbs results in enhanced apoptotic effects on BT474 breast tumor cells where anti-HER2 MAb 7C2 is administered prior to anti-HER2 MAb 4D5 (see Example 3).

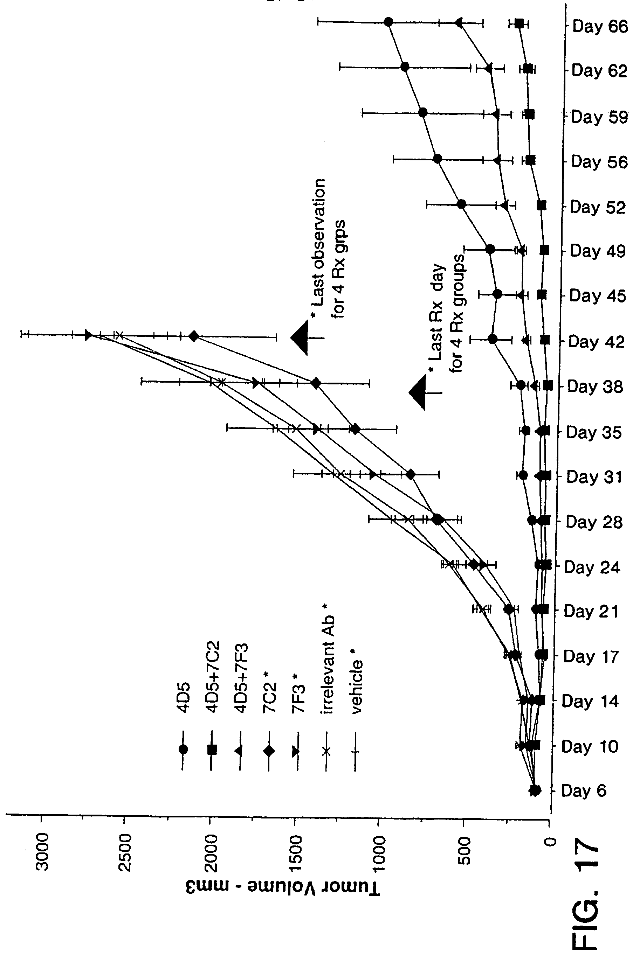

Fig. 17 shows the effects of administration of anti-HER2 MAbs alone or in combination on mean tumor volume (mm³) +/- 1 S.E. of BT474MI xenografts in nude mice as described in Example 4. Antibodies were administered twice weekly beginning on day 6.

### Detailed Description of the Preferred Embodiments

### 1. Definitions

Unless indicated otherwise, the term "ErbB2" when used herein refers to human ErbB2 protein and "*erb*B2" refers to human *erbB2* gene. The human *erbB2* gene and ErbB2 protein are described in Semba et al., PNAS (USA) 82:6497-6501 (1985) and Yamamoto et al. Nature 319:230-234 (1986) (Genebank accession number X03363), for example. ErbB2 comprises four domains (Domains 1-4). "Domain 1" at the amino terminus of the extracellular domain of ErbB2 is shown in Fig. 12 herein. See Plowman et al. Proc Natl. Acad. Sci USA 90:1746-1750 (1993).

The "epitope 7C2/7F3" is the region at the N terminus of the extracellular domain of ErbB2 to which the 7C2 and/or 7F3 antibodies (each deposited with the ATCC, see below) bind. To screen for antibodies which bind to the 7C2/7F3 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed (see Example 2 below) to establish whether the antibody binds to the 7C2/7F3 epitope on ErbB2 (i.e. any one or more of residues in the region from about residue 22 to about residue 53 of ErbB2 (SEQ ID NO:2)).

The "epitope 4D5" is the region in the extracellular domain of ErbB2 to which the antibody 4D5 (ATCC CRL 10463) binds. This epitope is close to the transmembrane region of ErbB2. To screen for antibodies which bind to the 4D5 epitope, a routine cross-blocking assay such as that described in Antibodies, A Laboratory Manual. Cold Spring Harbor Laboratory, Ed Harlow and David Lane (1988), can be performed. Alternatively, epitope mapping can be performed (see Example 2 below) to assess whether the antibody binds to the 4D5 epitope of ErbB2 (*i*.*e*. any one or more residues in the region from about residue 529, *e.g.* about residue 561 to about residue 625, inclusive (SEQ ID NO:4)).

The term "induces cell death" refers to the ability of the antibody to make a viable cell become nonviable. The "cell" here is one which expresses the ErbB2 receptor, especially where the cell overexpresses the ErbB2 receptor. A cell which "overexpresses" ErbB2 has significantly higher than normal ErbB2 levels compared to a noncancerous cell of the same tissue type. Preferably, the cell is a cancer cell, e.g. a breast, ovarian, stomach, endometrial, salivary gland, lung, kidney, colon, thyroid, pancreatic or bladder cell. *In vitro,* the cell may be a SKBR3. BT474, Calu 3, MDA-MB-453, MDA-MB-361 or SKOV3 cell. Cell death *in vitro* may be determined in the absence of complement and immune effector cells to distinguish cell death induced by antibody dependent cellular cytotoxicity (ADCC) or complement dependent cytotoxicity (CDC). Thus, the assay for cell death may be performed using heat inactivated serum (i.e. in the absence of complement) and in the absence of immune effector cells. To determine whether the antibody is able to induce cell death, loss of membrane integrity as evaluated by uptake of propidium iodide (PI) (see Example 2 below), trypan blue (see Moore et al. Cylotechnology 17:1-11 (1995)) or 7AAD (see Example 1 below) can be assessed relative to untreated cells. Preferred cell death-inducing antibodies are those which induce PI uptake in the "PI uptake assay in BT474 cells" (see below)

The phrase "induces apoptosis" refers to the ability of the antibody to induce programmed cell death as determined by binding of annexin V, fragmentation of DNA, cell shrinkage, dilation of endoplasmatic reticulum, cell fragmentation, and/or formation of membrane vesicles (called apoptotic bodies). See Figs. 1A & B herein. The cell is one which overexpresses the ErbB2 receptor. Preferably the "cell" is a tumor cell, *e.g.* a breast, ovarian, stomach. endometrial. salivary gland, lung, kidney, colon, thyroid, pancreatic or bladder cell. *In vitro,* the cell may be a SKBR3, BT474, Calu 3 cell, MDA-MB-453, MDA-MB-361 or SKOV3 cell. Various methods are available for evaluating the cellular events associated with apoptosis. For example, phosphatidyl serine (PS) translocation can be measured by annexin binding (see Example 2 below); DNA fragmentation can be evaluated through DNA laddering as disclosed in Example 2 herein: and nuclear/chromatin condensation along with DNA fragmentation can be evaluated by any increase in hypodiploid cells. Preferably, the antibody which induces apop'osis is one which results in about 2 to 50 fold, preferably about 5 to 50 fold, and most preferably about 10 to 50 fold, induction of annexin binding relative to untreated cell in an "annexin binding assay using BT474 cells" (see below).

Sometimes the pro-apoptotic antibody will be one which blocks HRG binding/activation of the ErbB2/ErbB3 complex (*e.g*. 7F3 antibody). In other situations, the antibody is one which does not significantly block activation of the ErbB2/ErbB3 receptor complex by HRG (*e.g* 7C2). Further, the antibody may be one like 7C2 which, while inducing apoptosis, does not induce a large reduction in the percent of cells in S phase (*e.g.* one which only induces about 0-10% reduction in the percent of these cells relative to control as determined in Fig. 10).

The antibody of interest may be one like 7C2 which binds specifically to human ErbB2 and does not significantly cross-react with other proteins such as those encoded by the *erb*B1*, erb*B3 and/or *erb*B4 genes. Sometimes, the antibody may not significantly cross-react with the rat *neu* protein, *e.g.,* as described in Schecter et al. Nature 312:513 (1984) and Drebin et al., Nature 312:545-548 (1984). In such embodiments; the extent of binding of the antibody to these proteins (*e.g*., cell surface binding to endogenous receptor) will be less than about 10% as determined by fluorescence activated cell sorting (FACS) analysis or radioimmunoprecipitation (RIA).

"Heregulin" (HRG) when used herein refers to a polypeptide which activates the ErbB2-ErbB3 and ErbB2-ErbB4 protein complexes (*i.e.* induces phosphorylation of tyrosine residues in the complex upon binding thereto). Various heregulin polypeptides encompassed by this term are disclosed in Holmes et al., Science, 256:1205-1210 (1992); WO 92/20798; Wen et al., Mol. Cell. Biol., 14(3):1909-1919 (1994); and Marchionni et al., Nature, 362:312-318 (1993), for example. The term includes biologically active fragments and/or variants of a naturally occurring HRG polypeptide, such as an EGF-like domain fragment thereof (*e.g*. HRGβI₁₁₇₋₂₄₄).

The "ErbB2-ErbB3 protein complex" and "ErbB2-ErbB4 protein complex" are noncovalently associated oligomers of the ErbB2 receptor and the ErbB3 receptor or ErbB4 receptor, respectively. The complexes form when a cell expressing both of these receptors is exposed to HRG and can be isolated by immunoprecipitation and analyzed by SDS-PAGE as described in Sliwkowski et al., J. Biol. Chem., 269(20):14661-14665 (1994).

"Antibodies" (Abs) and "immunoglobulins" (lgs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Native antibodies" and "native immunoglobulins" are usually heterotetrameric glycoproteins of about 150.000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light- chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat *et al., NIH Publ. No.91-3242,* Vol. 1, pages 647-669 (1991)). The constant domains are not involved directly in binding an antibody to an antigen, but exhibit various effector functions, such as participation of the antibody in antibody-dependent cellular toxicity.

Papain digestion of antibodies produces two identical antigen- binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen)-has the ability to recognize and bind antigen, although at a lower affinity than the entire binding site.

The Fab fragment also contains the constant domain of the light chain and the first constant domain (CH1) of the heavy chain. Fab fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g*., IgG1, IgG2, IgG3, IgG4, IgA, and IgA2. The heavy-chain constant domains that correspond to the different classes of immunoglobulins are called α. δ, ε, γ, and µ, respectively. The subunit structures and three-dimensional configurations of different classes of immunoglobulins are well known.

The term "antibody" is used in the broadest sense and specifically covers intact monoclonal antibodies, polyclonal antibodies, multispecific antibodies (*e.g*. bispecific antibodies) formed from at least two intact antibodies, and antibody fragments so long as they exhibit the desired biological activity.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al. Protein Eng. 8(10):1057-1062 (1995)); single-chain antibody molecules; and multispecific antibodies formed from antibody fragments.

The term "monoclonal antibody" as used herem refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature. 256:495 (1975), or may be made by recombinant DNA methods (see, *e.g.,* U.S. Patent No. 4,816,567). The "monoclonal antibodies" may also be isolated from phage antibody libraries using the techniques described in Clackson et al, Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad Sci. USA, 81:6851-6855 (1984)).

"Humanized" forms of non-human (*e g*., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZEDT™antibody wherein the antigen-binding region of the antibody is derived from an antibody produced by immunizing macaque monkeys with the antigen of interest.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH₎ connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

As used herein, the term "salvage receptor binding epitope" refers to an epitope of the Fc region of an IgG molecule (*e.g.,* IgG₁, IgG₂, IgG₃, or IgG₄) that is responsible for increasing the *in vivo* serum half-life of the IgG molecule.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, cats, cows, *etc* Preferably, the mammal is human.

A "disorder" is any condition that would benefit from treatment with the anti-ErbB2 antibody. This includes chronic and acute disorders or diseases including those pathological conditions which predispose the mammal to the disorder in question. Non-limiting examples of disorders to be treated herein include benign and malignant tumors; leukemias and lymphoid malignancies; neuronal, glial, astrocytal, hypothalamic and other glandular, macrophagal, epithelial, stromal and blastocoelic disorders; and inflammatory, angiogenic and immunologic disorders.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, liver cancer, prostate cancer, vulval cancer, thyroid cancer, hepatic carcinoma and various types of head and neck cancer.

The term "cytotoxic agent" as used herein refers to a substance that inhibits or prevents the function of cells and/or causes destruction of cells. The term is intended to include radioactive isotopes (*e.g.* I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include Adriamycin, Doxorubicin. 5-Fluorouracil, Cytosine arabinoside ("Ara-C"), Cyclophosphamide, Thiotepa, Busulfan, Cytoxin, Taxol. Toxotere, Methotrexate, Cisplatin, Melphalan, Vinblastine, Bleomycin, Etoposide, lfosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (see U.S. Pat. No. 4,675,187), Melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially an ErbB2-overexpressing cancer cell either *in vitro* or *in vivo,* Thus, the growth inhibitory agent is one which significantly reduces the percentage of ErbB2 overexpressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo [] inhibitors such as doxorubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al (WB Saunders: Philadelphia, 1995), especially p. 13. The 4D5 antibody (and functional equivalents thereof) can also be employed for this purpose.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide: inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-I, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

The word "label" when used herein refers to a detectable compound or composition which is conjugated directly or indirectly to the antibody so as to generate a "labelled" antibody. The label may be detectable by itself (*e.g.* radioisotope labels or fluorescent labels) or, in the case of an enzymatic label, may catalyze chemical alteration of a substrate compound or composition which is detectable.

By "solid phase" is meant a non-aqueous matrix to which the antibody can adhere. Examples of solid phases encompassed herein include those formed partially or entirely of glass (*e.g*., controlled pore glass), polysaccharides (*e*.*g*., agarose), polyacrylamides, polystyrene, polyvinyl alcohol and silicones. In certain embodiments, depending on the context, the solid phase can comprise the well of an assay plate; in others it is a purification column (*e g.,* an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as the anti-ErbB2 antibodies disclosed herein and, optionally, a chemotherapeutic agent) to a mammal. The components of the liposome are commonly arranged in a bilayer formation, similar to the lipid arrangement of biological membranes. An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokoryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription af the sequence; or a ribosome binding site is operably linked to a coding sequence if it is positioned so as to facilitate translation, Preferably, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell." "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

### II. Modes for Carrying out the Invention

### A. Antibody Preparation

A description follows as to exemplary techniques for the production of the claimed antibodies. The ErbB2 antigen to be used for production of antibodies may be, *e.g*., a soluble form of the extracellular domain of ErbB2; a peptide such as a Domain 1 peptide or a portion thereof (*e*.*g*. comprising the 7C2 or 7F3 epitope). Alternatively, cells expressing ErbB2 at their cell surface (*e.g*. NIH-3T3 cells transformed to overexpress ErbB2, see Examples 1 & 2 below; or a carcinoma cell line such as SKBR3 cells, see Stancovski et al. PNAS (USA) 88:8691-8695 (1991)) can be used to generate antibodies. Other forms of ErbB2 useful for generating antibodies will be apparent to those skilled in the art.

### (i) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g.,* keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g.,* 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (ii) Monoclonal antibodies

Monoclonal antibodies are obtained from a population of substantially homogeneous antibodies, *i.e*., the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or PPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al.. Curr. Opinion in Immunol., 5:256-262 (1993) and Plückthun, Immunol. Revs., 130:151-188 (1992).

In a further embodiment, antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy-and light-chain constant domains in place of the homologous murine sequences (U.S. Patent No. 4.816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized and human antibodies

Methods for humanizing non-human antibodies are well known in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al, Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immnol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggermann et al., Year in Immuno., 7:33 (1993). Human antibodies can also be derived from phage-display libraries (Hoogenboom et al., J. Mol. Biol., 227:381 (1991); Marks et al., J. Mol. Biol., 222:581-597 (1991)).

### (iv) Antibody fragments

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185.

### (v) Bispecific antibodies

Bispecific antibodies are antibodies that have binding specificities for at least two different epitopes. Exemplary bispecific antibodies may bind to two different epitopes of the ErbB2 protein. For example, one arm may bind an epitope in Domain I of ErbB2 such as the 7C2/7F3 epitope, the other may bind a different ErbB2 epitope, e.g. the 4D5 epitope. Other such antibodies may combine an ErbB2 binding site with binding site(s) for EGFR, ErbB3 and/or ErbB4. Alternatively, an anti-ErbB2 arm may be combined with an arm which binds to a triggering molecule on a leukocyte such as a T-cell receptor molecule (*e.g.* CD2 or CD3), or Fc receptors for IgG (FcγR), such as FcγRI (CD64), FcγRII (CD32) and FcγRIII (CD16) so as to focus cellular defense mechanisms to the ErbB2-expressing cell. Bispecific antibodies may also be used to localize cytotoxic agents to cells which express ErbB2. These antibodies possess an ErbB2-binding arm and an arm which binds the cytotoxic agent (*e.g.* saporin, anti-interferon-α, vinca alkaloid, ricin A chain, methotrexate or radioactive isotope hapten). Bispecific antibodies can be prepared as full length antibodies or antibody fragments (*e.g*. F(ab')₂ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in WO96/27011, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant, cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al. J. Immunol., 152:5368 (1994).

Antibodies with more than two valencies are contemplated. For example, trispecific antibodies can be prepared. Tutt et al. J. Immunol. 147: 60 (1991).

### (vi) Screening for antibodies with the desired properties

Techniques for generating antibodies have been described above. Those antibodies having the characteristics described herein are selected.

To select for antibodies which induce cell death, loss of membrane integrity as indicated by, *e.g.,* PI, trypan blue or 7AAD uptake is assessed relative to control. The preferred assay is the "PI uptake assay using BT474 cells". According to this assay, BT474 cells (which can be obtained from the American Type Culture Collection (Rockville, MD)) are cultured in Dulbecco's Modified Eagle Medium (D-MEM):Ham's F-12 (50:50) supplemented with 10% heat-inactivated FBS (Hyclone) and 2 mM L-glutamine. (Thus, the assay is performed in the absence of complement and immune effector cells). The BT474 cells are seeded at a density of 3 x 10⁶ per dish in 100 x 20 mm dishes and allowed to attach overnight. The medium is then removed and replaced with fresh medium alone or medium containing 10µg/ml of the appropriate MAb. The cells are incubated for a 3 day time period. Following each treatment, monolayers are washed with PBS and detached by trypsinization. Cells are then centrifuged at 1200rpm for 5 minutes at 4⁰C, the pellet resuspended in 3 ml ice cold Ca²⁺ binding buffer (10 mM Hepes, pH 7.4, 140 mM NaCl, 2.5 mM CaCl₂) and aliquoted into 35 mm strainer-capped 12 x 75 tubes (1ml per tube, 3 tubes per treatment group) for removal of cell clumps. Tubes then receive PI (10µg/ml). Samples may be analyzed using a FACSCAN™ flow cytometer and FACSCONVERT™ CellQuest software (Becton Dickinson). Those antibodies which induce statistically significant levels of cell death as determined by PI uptake are selected.

In orderto select for antibodies which induce apoptosis, an "annexin binding assay using BT474 cells" as described in Example 2 below is available. The BT474 cells are cultured and seeded in dishes as discussed in the preceding paragraph. The medium is then removed and replaced with fresh medium alone or medium containing 10µg/ml of the MAb. Following a three day incubation period, monolayers are washed with PBS and detached by trypsinization. Cells are then centrifuged, resuspended in Ca²⁺ binding buffer and aliquoted into tubes as discussed above for the cell death assay. Tubes then receive labelled annexin (*e.g*. annexin V-FTIC) (1µg/ml). Samples may be analyzed using a FACSCAN™ flow cytometer and FACSCONVERT™ CellQuest software (Becton Dickinson). Those antibodies which induce statistically significant levels of annexin binding relative to control are selected as apoptosis-inducing antibodies.

In addition to the annexin binding assay discussed in the preceding paragraph, a "DNA staining assay using BT474 cells" is available. In order to perform this assay, BT474 cells which have been treated with the antibody of interest as described in the preceding two paragraphs are incubated with 9µg/ml HOECHST 33342™ for 2 hr at 37⁰C, then analyzed on an EPICS ELITE™ flow cytometer (Coulter Corporation) using MODFIT LT™ software (Verity Software House). Antibodies which induce a change in the percentage of apoptotic cells which is 2 fold or greater (and preferably 3 fold or greater) than untreated cells (up to 100% apoptotic cells) may be selected as pro-apoptetic antibodies using this assay.

To screen for antibodies which bind to an epitope on ErbB2 bound by an antibody of interest, a routine cross-blocking assay such as that described in Antibodies. A Laboratory Manual, Cold Spring Harbor Laboratory, Ed Harlow and David Lane (1988), can be performed. Alternatively, epitope mapping described in Example 2 can be performed.

To identify anti-ErbB2 antibodies which inhibit growth of SKBR3 cells in cell culture by 50-100%, the SKBR3 assay described in WO89/06692 can be performed. According to this assay, SKBR3 cells are grown in a 1:1 mixture of F12 and DMEM medium supplemented with 10% fetal bovine serum, glutamine and penicillinstreptomycin. The SKBR3 cells are plated at 20,000 cells in a 35mm cell culture dish (2mls/35mm dish). 2.5µg/ml of the anti- ErbB2 antibody is added per dish. After six days, the number of cells, compared to untreated cells are counted using an electronic COULTER™ cell counter. Those antibodies which inhibit growth of the SKBR3 cells by 50-100% are selected for combination with the apoptotic antibodies as desired.

### (vii) Effector function engineering

It may be desirable to modify the antibody with respect to effector function, so as to enhance the effectiveness of the antibody in treating cancer, for example. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J Exp Med. 176:1191-1195 (1992) and Shopes, B. J. Immunol 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

### (viii) Immunoconjugates

The invention also pertains to immunoconjugates comprising the antibody described herein conjugated to a cytotoxic agent such as a chemotherapeutic agent, toxin (*e.g.* an enzymatically active toxin of bacterial, fungal, plant or animal origin, or fragments thereof), or a radioactive isotope (*i.e.*, a radioconjugate).

Chemotherapeutic agents useful in the generation of such immunoconjugates have been described above. Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crolin, sapaonaria officinalis inhibitor, gelonin, mitogellin, restrictocin, phenomycin, enomycin and the tricothecenes. A variety of radionuclides are available for the production of radioconjugated anti-ErbB2 antibodies. Examples include ²¹²Bi, ¹³¹I, ¹³¹In, ⁹⁰Y and ¹⁸⁶Re.

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2.6-diisocyanate), and bis-active fluorine compounds (such as 1.5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g*. avidin) which is conjugated to a cytotoxic agent (*e.g*. a radionucleotide).

### (ix) Immunoliposomes

The anti-ErbB2 antibodies disclosed herein may also be formulated as immunoliposomes. Liposomes containing the antibody are prepared by methods known in the art, such as described in Epstein et al. Proc. Natl. Acad. Sci. USA, 82:3688 (1985); Hwang et al., Proc. Natl Acad. Sci. USA, 77:4030 (1980); and U.S. Pat. Nos. 4,485,043 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody can be conjugated to the liposomes as described in Martin et al. J. Biol. Chem. 257: 286-288 (1982) via a disulfide interchange reaction. A chemotherapeutic agent (such as Doxorubicin) is optionally contained within the liposome. See Gabizon et al J National Cancer Inst. 81(19)1484 (1989)

### (x) Antibody Dependent Enzyme Mediated Prodrug Therapy (ADEPT)

The antibody may also be used in ADEPT by conjugating the antibody to a prodrug-activating enzyme which converts a prodrug (*e.g.* a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substiments; carbohydrate-cleaving enzymes such as ß-galactosidase and neuraminidase useful for converting glycosylated prodrugs into free drugs; β-lactamase useful for convening drugs derivatized with β-lactams into free drugs; and penicillin amidases, such as penicillin V amidase or penicillin G amidase, useful for convening drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs into free active drugs (see, *e*.*g*., Massey, Nature 328: 457-458 (1987)). Antibody-abzyme conjugates can be prepared as described herein for delivery of the abzyme to a tumor cell population.

The enzymes can be covalently bound to the anti-ErbB2 antibodies by techniques well known in the an such as the use of the heterobifunctional crosslinking reagents discussed above. Alternatively, fusion proteins comprising at least the antigen binding region of an antibody linked to at least a functionally active portion of an enzyme can be constructed using recombinant DNA techniques well known in the art (see, *e.g*., Neuberger et al., Nature, 312:604-608 (1984)).

### (xi) Antibody-salvage receptor binding epitope fusions.

In certain embodiments of the invention, it may be desirable to use an antibody fragment, rather than an intact antibody, to increase tumor penetration, for example. In this case, it may be desirable to modify the antibody fragment in order to increase its serum half life. This may be achieved, for example, by incorporation of a salvage receptor binding epitope into the antibody fragment (*e.g*. by mutation of the appropriate region in the antibody fragment or by incorporating the epitope into a peptide tag that is then fused to the antibody fragment at either end or in the middle, *e.g*., by DNA or peptide synthesis).

A systematic method for preparing such an antibody variant having an increased *in vivo* half-life comprises several steps. The first involves identifying the sequence and conformation of a salvage receptor binding epitope of an Fc region of an IgG molecule. Once this epitope is identified, the sequence of the antibody of interest is modified to include the sequence and conformation of the identified binding epitope. After the sequence is mutated, the antibody variant is tested to see if it has a longer *in vivo* half-life than that of the original antibody. If the antibody variant does not have a longer *in vivo* half-life upon testing, its sequence is further altered to include the sequence and conformation of the identified binding epitope. The altered antibody is tested for longer *in vivo* half-life, and this process is continued until a molecule is obtained that exhibits a longer *in vivo* half-life.

The salvage receptor binding epitope being thus incorporated into the antibody of interest is any suitable such epitope as defined above, and its nature will depend, *e.g.,* on the type of antibody being modified. The transfer is made such that the antibody of interest still possesses the biological activities described herein.

The epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (*e*.*g*., of an IgG) and transferred to the CH1, CH3, or V_{H} region, or more than one such region, of the antibody. Alternatively, the epitope is taken from the CH2 domain of the Fc region and transferred to the C_{L} region or V_{L} region, or both, of the antibody fragment.

In one most preferred embodiment, the salvage receptor binding epitope comprises the sequence (5' to 3'): PKNSSMISNTP (SEQ ID NO:5), and optionally further comprises a sequence selected from the group consisting of HQSLGTQ (SEQ ID NO:6), HQNLSDGK (SEQ ID NO:7), HQNISDGK (SEQ ID NO:8), or VISSHLGQ (SEQ ID NO:9), particularly where the antibody fragment is a Fab or F(ab')₂. In another most preferred embodiment, the salvage receptor binding epitope is a polypeptide containing the sequence(s)(5' to 3'): HQNLSDGK (SEQ ID NO:7), HQNISDGK (SEQ ID NO:8), or VISSHLGQ (SEQ ID NO:9) and the sequence: PKNSSMISNTP (SEQ ID NO:5).

### B. Vectors, Host Cells and Recombinant Methods

For recombinant production of the antibody, the nucleic acid encoding it is isolated and inserted into a replicable vector for further cloning (amplification of the DNA) or for expression. DNA encoding the monoclonal antibody is readily isolated and sequenced using conventional procedures (*e.g*., by using oligonucleotide probes that are capable of binding specifically to genes encoding the heavy and light chains of the antibody). Many vectors are available. The vector components preferably include, but are not limited to, one or more of the following: a signal sequence, an origin of replication, one or more marker genes, an enhancer element, a promoter, and a transcription termination sequence.

### (i) Signal sequence component

The anti-ErbB2 antibody may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (*i.e*., cleaved by a signal peptidase) by the host cell. For prokaryotic host cells that do not recognize and process the native anti-ErbB2 antibody signal sequence, the signal sequence is substituted by a prokaryotic signal sequence selected, for example, from the group of the alkaline phosphatase, penicillinase, Ipp, or heat-stable enterotoxin II leaders. For yeast secretion the native signal sequence may be substituted by, *e.g*., the yeast invertase leader, α factor leader (including *Saccharomyces* and *Kluyverοmyces* α-factor leaders), or acid phosphatase leader, the C *albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the anti-ErbB2 antibody.

### (ii) Origin of replication component

Both expression and cloning vectors contain a nucleic acid sequence that enables the vector to replicate in one or more selected host cells. Preferably, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Preferably, the origin of replication component is not needed for mammalian expression vectors (the SV40 origin may typically be used only because it contains the early promoter).

### (iii) Selection gene component

Expression and cloning vectors may contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolie acid and hygromycin.

Another example of suitable selectable markers for mammalian cells are those that enable the identification of cells competent to take up the anti-ErbB2 antibody nucleic acid, such as DHFR, thymidine kinase, metallothionein-I and -II, preferably primate metallothionein genes, adenosine deaminase, ornithine decarboxylase, *etc.*

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding anti-ErbB2 antibody, wild-type DHFR protein, and another selectable marker such as aminoglycoside 3'-phosphotransferase (APH) can be selected by cell growth in' medium containing a selection agent for the selectable marker such as an aminoglycosidic antibiotic, *e.g*, kanamycin, neomycin, or G418. See U.S. Patent No. 4,965,199.

A suitable selection gene for use in yeast is the *trip*1 gene present in the yeast plasmid YRp7 (Stinchcomb et al., Nature, 282:39 (1979)). The *trp*1 gene provides a selection marker for a mutant strain of yeast lacking the ability to grow in tryptophan, for example, ATCC No. 44076 or PEP4-1. Jones, Genetics, 85:12 (1977). The presence of the *trp*1 lesion in the yeast host cell genome then provides an effective environment for detecting transformation by growth in the absence of tryptophan. Similarly, *Leu*2-deficient yeast strains (ATCC 20,622 or 38.626) are complemented by known plasmids bearing the *Leu*2 gene.

In addition, vectors derived from the 1.6µm circular plasmid pKDI can be used for transformation; of *Kluyveromyces* yeasts. Alternatively, an expression system for large-scale production of recombinant calf chymosin was reported for *K. lactis.* Van den Berg, Bio/Technology, 8:135 (1990). Stable multi-copy expression vectors for secretion of mature recombinant human serum albumin by industrial strains of *Kluyveromyces* have also been disclosed. Fleer et al., Bio/Technology, 9:968-975 (1991).

### (iv) Promoter component

Expression and cloning vectors usually contain a promoter that is recognized by the host organism and is operably linked to the anti-ErbB2 antibody nucleic acid. Promoters suitable for use with prokaryotic hosts include the *phoA* promoter , β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the anti-ErbB2 antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, giyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism. metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-ErbB2 antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, *e.g*., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature, 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### (v) Enhancer element component

Transcription of a DNA encoding the anti-ErbB2 antibody by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of then replication origin (bp 100-270), the cytomegalovirus early promoter enhancer, the polyoma enhancer on the late side of the replication origin, and adenovirus enhancers. See also Yaniv, Nature, 297:17-18 (1982) on enhancing elements for activation of eukaryotic promoters. The enhancer may be spliced into the vector at a position 5' or 3' to the anti-ErbB2 antibody-encoding sequence, but is preferably located at a site 5' from the promoter.

### (vi) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucteotide segments transcribed as polyadenylated fragments in the untranslated portion of the mRNA encoding anti-ErbB2 antibody. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia*, *e*.*g*., *E. coli*, *Enterobacter, Erwinia, Klebsiella*, *Proteus, Salmonella, e.g., Salmonella typhimurium, Serratia, e*.*g*., *Serratia marcescans,* and *Shigella,* as well as *Bacilli* such as *B. subtilis* and *B. licheniformis* (*e*.*g*., *B*. *licheniformis* 41 P. disclosed in DD 266,710 published 12 April 1989), *Pseudomonas such* as *P. aeruginosa,* and *Streptomyces*. One preferred *E.* colt cloning host is *E. coli* 294 (ATCC, 31,446), although other strains such as *E. coli B*, *E*. *coli* X1776 (ATCC 31,537), and *E. coli* W3310 (ATCC 27,325) are suitable. These examples are illustrative rather than limiting.

In addition to prokaryotes, eukaryotic microbes such as filamentous fungi or yeast are suitable cloning or expression hosts for anti-ErbB2 antibody-encoding vectors. *Saccharomyces cerevisiae*, or common baker's yeast, is the most commonly used among lower eukaryotic host microorganisms. However, a number of other genera, species, and strains are commonly available and useful herein, such as *Schizosaccharomyces pombe*: *Kluyveromyces* hosts such as, *e.g.*, *K. lactis. K*. *fragilis* (ATCC, 12.424), *K. bulgaricus* (ATCC 16,045), *K. wickeramii* (ATCC 24,178), *K. waltii (*ATCC 56,500), *K*. *drosaphilarum* (ATCC 36,906), *K. thermotolerans,* and *K. marxianus*; *yarrowia* (EP 402,226); *Pichia pastoris* (EP 183,070); *Candida*; *Trichoderma reesia* (EP 244.234); *Neurospora crassa*; *Schwanniomyces* such *as Schwanniomyces occidentalis*; and filamentous fungi such as, *e.g.*, *Neurospora, Penicillium, Tolypocladium*, and *Aspergillus* hosts such as *A. nidulans* and *A*. *niger.*

Suitable host cells for the expression of glycosylated anti-ErbB2 antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, *e*.*g*., the L-1 variant of *Autographa californica* NPV and the Bm-5 strain of *Bombyx mori* NPV, and such viruses may be used, particularly for transfection of *Spodoptera frugiperda* cells.

Plant cell cultures of cotton, com, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CVI line transformed by SV40 (COS-7, ATCC, CRL 1651); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells/-DHFR (CHO, Urlaub *et al*., *Proc*. *Natl. Acad. Sci.* USA, 77:4216 (1980)); mouse sertoli cells (TM4, Mather, Biol. Reprod., 23:243-251 (1980)); monkey kidney cells (CV1 ATCC, CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC, CCL51); TRI cells (Mather et al., Annals N. Y. Acad Sci., 383:44-68 (1982)); MRC 5 cells; FS4 cells; and a human hepatoma line (Hep G2).

Host cells are transformed with the above-described expression or cloning vectors for anti-ErbB2 antibody production and cultured in conventional nutrient media modified as appropriate for inducing promoters, selecting transformants, or amplifying the genes encoding the desired sequences.

### (viii) Culturing the host cells

The host cells used to produce the anti-ErbB2 antibody may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al. Meth. Enz., 58:44 (1979), Barnes et al., Anal. Biochem., 102:255 (1980), U.S. Pat. Nos. 4,767,704; 4,657,866, 4,927,762; 4,560,655; or 5,122,469; WO 90/03430; WO 87/00195: or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of anti-ErbB2 antibody

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are preferably first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H}3 domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e*.*g*. from about 0-0.25M salt).

### C. Pharmaceutical Formulations '

Therapeutic formulations of the antibody are prepared for storage by mixing the antibody having the desired degree of purity with optional pharmaceutically acceptable carriers, excipients or stabilizers' (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride: benzalkonium chloride, benzethonium chloride: phenol, butyl or benzyl alcohol; alkyl parabens such as methyl or propyl paraben: catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine: monosaccharides, disaccharides, and other carbohydrates including glucose, mannose, or dextrins, chelating agents such as EDTA; sugars such as sucrose, mannitol, trehalose or sorbitol; salt-forming counter-ions such as sodium; metal complexes (*e*.*g*. Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. For example, it may be desirable to further provide antibodies which bind to EGFR, ErbB2 (*e*.*g*. an antibody which binds a different epitope on ErbB2), ErbB3, ErbB4. or vascular endothelial factor (VEGF) in the one formulation. Alternatively, or in addition, the composition may comprise a cytotoxic agent, cytokine or growth inhibitory agent. Such molecules are suitably present in combination in amounts that are effective for the purpose intended.

The active ingredients may also be entrapped in microcapsules prepared, for example, by coacervation techniques or by interfacial polymerization, for examples, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e*.*g*. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™(injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through this-disulfide interchange, stabilization may be achieved by modifying sulfhydryl residues, lyophilizing from acidic solution. controlling moisture content, using appropriate additives, and developing specific polymer matrix compositions.

### D. Therapeutic Uses for the Antibody

Exemplary conditions or disorders to be treated with the ErbB2 antibody include benign or malignant tumors (*e*.*g*. renal, liver, kidney, bladder, breast, gastric, ovarian, colorectal, prostate, pancreatic, ling, vulval, thyroid, hepatic carcinomas; sarcomas; glioblastomas; and various head and neck tumors); leukemias and lymphoid malignancies: other disorders such as neuronal, glial, astrocytal, hypothalamic and other glandular, macrophagal, epithelial stromal and blastocoelic disorders; and inflammatory, angiogenic and immunologic disorders.

The antibodies are to be administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Other therapeutic regimens may be combined with the administration of the anti-ErbB2 antibodies. For example, the patient to be treated with the antibodies disclosed herein may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the antibody or may be given simultaneously therewith. The antibody may be combined with an anti-oestrogen compound such as tamoxifen or an anti-progesterone such as onapristone (see, EP 616812) in dosages known for such molecules.

It may be desirable to also administer antibodies against other tumor associated antigens, such as antibodies which bind to the EGFR, ErbB3, ErbB4, or vascular endothelial factor (VEGF). Alternatively, or in addition, two or more anti-ErbB2 antibodies may be co-administered to the patient. Sometimes, it may be. beneficial to also administer one or more cytokines to the patient. In a preferred embodiment, the ErbB2 antibody is co-administered with a growth inhibitory agent. For example, the growth inhibitory agent may be administered first, followed by the ErbB2 antibody. However, simultaneous administration or administration of the ErbB2 antibody first is also contemplated. Suitable dosages for the growth inhibitory agent are those presently used and may be lowered due to the combined action (synergy) of the growth inhibitory agent and anti-ErbB2 antibody.

For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments

Depending on the type and severity of the disease, about 1 µg/kg to 15 mg/kg (*e*.*g*. 0.1-20m/kg) of antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs However, other dosage regimens may be useful. The progress of this therapy is easily monitored by conventional techniques and assays.

### E. Deposit of Materials

The following hybridoma cell lines have been deposited with the American Type Culture Collection, 12301 Parklawn Drive, Rockville, MD, URSA (ATCC):

| **Antibody Designation** | **ATCC No.** | **Deposit Date** |
|---|---|---|
| 7C2 | ATCC HB-12215 | October 17, 1996 |
| 7F3 | ATCC HB-12216 | October 17, 1996 |
| 4D5 | ATCC CRL 10463 | May 24, 1990 |

These deposits were made under the provisions of the Budapest Treaty on the International Recognition of the Deposit of Microorganisms for the Purpose of Patent Procedure and the Regulations thereunder (Budapest Treaty). This assures maintenance of viable cultures for 30 years from the date of deposit. The cell lines will be made available by ATCC under the terms of the Budapest Treaty, and subject to an agreement between Genentech, Inc. and ATCC, which assures (a) that access to the cultures will be available during pendency of the patent application to one determined by the Commissioner to be entitled thereto under 37 CFR §1.14 and 35 USC §122. and (b) that all restrictions on the availability to the public of the cultures so deposited will be irrevocably removed upon the granting of the patent.

The assignee of the present application has agreed that if the cultures on deposit should die or be lost or destroyed when cultivated under suitable conditions, they will be promptly replaced on notification with a viable specimen of the same cultures. Availability of the deposited cell lines is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the antibodies deposited, since any antibody that is functionally equivalent is within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents. Indeed, various modifications of the invention in addition to those shown and described herein will become apparent to those skilled in the art from the foregoing description and fall within the scope of the appended claims.

The following examples are offered by way of illustration and not by way of limitation.

### EXAMPLE 1

### INDUCTION OF CELL DEATH

Cell lines. The established human breast tumor cells BT474 and MDA-MB-231 (which are available from (ATCC) were grown in minimum essential medium (Gibco, Grand Island, NY) supplemented with 10% heat-inactivated fetal bovine serum (FBS) (HyClone, Logan, UT), sodium pyruvate. L-glutamine (2mM), non-essential amino acids and 2x vitamin solution and maintained at 37°C in 5% CO₂ (Zhang et al. Invas. & Metas. 11(4):204-215 (1991) and Price et al Cancer Res. 50(3):717-721 (1990)).

Antibodies. The anti-ErbB2 IgG₁κ murine monoclonal antibodies 4D5 and 7C2, specific for the extracellular domain of ErbB2, were produced as described in Fendly et al Cancer Research 50:1550-1558 (1990) and WO89/06692. Briefly, NIH 3T3/HER2-3₄₀₀ cells (expressing approximately 1 x 10⁵ ErbB2 molecules/cell) produced as described in Hudziak et al. Proc, Natl Acdd. Sci. (USA) 84:7159 (1987) were harvested with phospate buffered saline (PBS) containing 25mM EDTA and used to immunize BALB/c mice. The mice were given injections i.p. of 10⁷ cells in 0.5ml PBS on weeks, 0,2,5 and 7. The mice with antisera that immunoprecipitated ³²P-labeled ErbB2 were given i.p. injections of a wheat germ agglutinin-Sepharose (WGA) purified ErbB2 membrane extract on weeks 9 and 13. This was followed by an i.v. injection of 0.1ml of the ErbB2 preparation and the splenocytes were fused with mouse myeloma line X63-Ag8.653. Hybridoma supernatants were screened for ErbB2-binding by ELISA and radioimmunoprecipitation. MOPC-21 (IgG1), (Cappell, Durham, NC), was used as an isotype-matched control.

Analysis of cell cycle status and viability. Cells were simultaneously examined for viability and cell cycle status by flow cytometry on a FACSTAR PLUS™ (Becton Dickinson Immunocytometry Systems USA. San Jose, CA). Breast tumor cells were harvested by washing the monolayer with phosphate buffered saline (PBS), incubating cells in 0.05% trypsin and 0.53 mM EDTA (Gibco) and resuspending them in culture medium. The cells were washed twice with PBS containing 1% FBS and the pellet was incubated for 30 minutes on ice with 50µl of 400µM 7 amino actinomycin D (7AAD) (Molecular Probes, Eugene, OR), a vital dye which stains all permeable cells. Cells were then fixed with 1.0ml of 0.5% paraformaldehyde in PBS and simultaneously permeabilized and stained for 16 hours at 4°C with 220µl of 10µg/ml HOECHST 33342™ dye (also a DNA binding dye) containing 5% TWEEN 20™.

The data from 1 x 10⁴ cells were collected and stored using LYSYS II™ software and analyzed using PAINT-A-GATE™ software (Becton Dickinson) (Darzynkiewica et al. Cytometry 13:795-808 (1992) and Picker et al. J. Immunol. 150(3):1105-1121 (1993)). The viability and percentage of cells in each stage of the cell cycle were determined on gated single cells using 7AAD and Hoechst staining, respectively. (Cell doublets were excluded by pulse analysis of width vs. area of the Hoechst signal.) Cell numbers were determined using a hemocytometer.

DNA synthesis. Triplicate cultures of 8 x 10³ cells/well were plated in 96-well flat bottom plates, allowed to adhere overnight, then continuously incubated in the presence or absence of anti-ErbB2 or control Ig for different periods of time. During the last 12 hours of culture, wells were pulsed with 1µCi³H-thymidine (Amersham, Arlington, VA).

Affinity of binding to the extracellular domain of the ErbB2. Hadioiodinated anti-ErbB2 antibodies were prepared by the lodogen method (Fracker et al. Biochem. Biophys. Res. Comm. 80:849-857 (1978)). Binding assays were performed using monolayers of BT474 cells cultured in 96-well tissue culture plates (Falcon, Becton Dickenson Labware, Lincoln Park, N.J.). The cells were trypsinized and seeded in wells of 96-well plates at a density of 10⁴ cells/well and allowed to adhere overnight. The monolayers were washed with cold culture medium supplemented with 0.1% sodium azide and then incubated in triplicate with 100µl of serial dilutions of ¹²⁵I-anti-ErbB2 antibodies in cold culture medium with 0.1% azide for 4 hours on ice. Non-specific binding was estimated by the preincubation of each sample with a 100-fold molar excess of nonradioactive antibodies in a total volume of 100µl. Unbound radioactivity was removed by two washes with cold medium with 0.1 % sodium azide. The cell-associated radioactivity was detected in a gamma counter after solubilization of the cells with 150µl 0.1 M NaOH/well. The anti-ErbB2 binding constants (*K*_{d}) were determined by Scatchard analysis.

Results. The binding affinities of anti-ErbB2 antibodies (7C2 and 4D5) were determined by Scatchard analysis. The binding constants (*K*_{d}) were 6.5 x 10⁻⁹ M (4D5) and 2.9 x 10⁻⁹ M (7C2). Blocking experiments were carried out using unlabelled antibodies followed by FITC-7C2. As shown in Fig. 2, 4D5 reacts with a different epitope than 7C2.

The effect of these antibodies on the growth of the BT474 human breast cancer cells which overexpress ErbB2 was then investigated. Fig. 3A shows the results of flow cytometric analysis of cells incubated with an isotype-matched control. 10-12% of the cells were dead and 28% of the viable cells were in the S-G₂-M phases of the cell cycle. Similar results were obtained when the cells were incubated in medium alone. Treatment with 4D5 (Fig. 3B) induced a decrease in cell size as measured by forward light scatter, a moderate increase in the proportion of dead cells (27.0%) and a marked decrease of viable cells in S-G₂-M (6.3%) with a concurrent increase of cells in G₀/G₁ (94%) as compared to the control cells. Cell counts were reduced by 46.7%. Without being bound to any one theory, it appears that 4D5 induces primarily cell cycle arrest (CCA) in G₀/G₁ but that a significant proportion of cells also die. Fig. 3C shows the results of incubating the BT474 cell with 50µg/ml of 7C2. There was no change in forward light scatter of the residual viable cells, a marked increase in the proportion of dead cells (72%) and a 70% decrease in cell count compared to control cells (data not shown). Hence, viable cells were decreased by 85%. There was a slight reduction in cycling cells (21% vs. an average of 29% for controls) but because of extensive cell death, it was difficult to distinguish CCA from a preferential loss of cycling cells. Hence, 7C2 and 4D5 affect cells differently; 7C2 induces predominantly cell death while 4D5 induces predominantly CCA. Fig. 3D shows the results of adding 50µg/ml 7C2 and 1µg/ml 4D5 to BT474 cells simultaneously. There was no increase in the proportion of dead cells compared to cells treated with 7C2 alone (Fig. 3C). However, the number of residual viable cells was reduced by an additional 50%. In addition, there was a marked increase in cells having both permeable membranes and significantly degraded DNA (<1 X). An analysis of the small number of residual viable cells showed that there was a similar reduction in cycling cells compared to cells treated with 4D5 alone (Fig. 3D compared to Fig. 3B).

As an additional control, MDA-MB-231 breast cancer cells, which express normal levels of ErbB2 (Lewis et al. Cancer Immunol. Immunther. 37:255-263 (1993)), were treated with 4D5 or 7C2. As compared to the control, neither antibody affected the growth of these cells (27-28% of viable cells in S-G₂-M and 12-13% dead cells).

The additive effects of both antibodies was clearly demonstrated when a suboptimal dose of 4D5 (0.05 µg/ml) was used. Fig. 4A shows that thymidine incorporation was reduced by 22.3% and 23% in cells treated with 7C2 and 4D5 respectively, and by 58% in cells treated with 4D5 and 7C2. An additional experiment utilizing 20µg 7C2 and 0.1µg 4D5 gave similar results, *i.e.,* thymidine incorporation was reduced by 41%, 25% and 72% in cells treated with 7C2 alone, 4D5 alone, or 4D5 plus 7C2, respectively. In Fig. 4B, the viable cell counts are shown. A total cell count was determined and FACS analysis was used to establish the number of viable cells. Viable cell counts were reduced by 64%, 29% and 84% in cells treated with 7C2, 4D5 or the combination, respectively, when compared to untreated controls.

### EXAMPLE 2

### INDUCTION OF APOPTOSIS

**Materials and cell culture.** All tumor cell lines were obtained from the American Type Culture Collection (Rockville, MD). Cells were cultured in Dulbecco's Modified Eagle Medium (D-MEM):Ham's F-12 (50:50) supplemented with 10% heat-inactivated fetal bovine serum (FBS) (Hyclone) and 2 mM L-glutamine. Human mammary epithelial cells (HMEC) were obtained from Clonetics and grown in MEGM (mammary epithelial growth medium, Clonetics) containing bovine pituitary extract. Biochemicals used were: annexin V-FTIC (BioWhittaker, Inc.), propidium iodide (PI, Molecular Probes, Inc.), and HOECHST 33342™ (Calbiochem). Anti-ErbB2 monoclonal antibodies (MAbs) were produced as described in Fendly et al. Cancer Research 50:1550-1558 (1990) and WO89/06692 (see Example 1 above). The anti-ErbB2 MAbs tested are designated: 4D5, 7C2, 7F3, 3H4, 2C4, 2H11, 3E8, and 7D3. The isotype-matched control MAb 1766 is directed against the herpes simplex virus (HSV-1) glycoprotein D.

**Flow cytometry experiments for measuring induction of apoptosis.** Cells were seeded at a density of 3 x 10⁶ per dish in 100 x 20 mm dishes and allowed to attach overnight. The medium was then removed and replaced with fresh medium alone or medium containing 10µg/ml of the appropriate MAb. For most experiments, cells were incubated for a 3 day time period. For time course studies, cells were treated for 0.25, 0.5, 1, 2, 24, 72, 96 hr, 7 or 10 days. MAb concentrations used in the dose-response experiments were 0.01, 0.1, 1 and 10µg/ml. Following each treatment, supernatants were individually collected and kept on ice, monolayers were detached by trypsinization and pooled with the corresponding supernatant. Cells were then centrifuged at 1200rpm for 5 minutes at 4°C, the pellet resuspended in 3 ml ice cold Ca²⁺ binding buffer (10 mM Hepes, pH 7.4, 140 mM NaCl, 2.5 mM CaCl₂) and aliquoted into 35 mm strainer-capped 12 x 75 tubes (1ml per tube, 3 tubes per treatment group) for removal of cell aggregates. Each group of 3 tubes then received annexin V-FTIC (1µg/ml) or PI (10µg/ml) or annexin V-FTIC plus PI. Samples were analyzed using a FACSCAN™ flow cytometer and FACSCONVERT™ CellQuest software (Becton Dickinson). For cell cycle analysis, cells were incubated with 9µg/ml HOECHST 33342™ for 2 hr at 37°C, then analyzed on an EPICS ELITE™ flow cytometer (Coulter Corporation) using MODFIT LT™ software (Verity Software House).

Serum-deprivation experiments were performed in the following way. BT474 breast tumor cells were seeded in culture medium at a density of 5 x 10⁶ per dish in 100 x 20 mm dishes. The following day, the medium was replaced with medium containing 0.1% FBS and the cells were incubated for 3 days. Cells then received 10µg/ml of MAb 7C2 or 4D5 in fresh medium supplemented with 0.1% FBS. After a 3 day incubation, analyses of annexin V binding, PI uptake and cell cycle progression were performed as described above. In order to compare growth of serum-starved cells to non-deprived cells, separate dishes of BT474 cells, incubated in medium supplemented with 10% FBS for all time points, were studied in parallel.

**Detection of DNA ladder formation.** For measuring internucleosomal fragmentation of DNA, BT474 breast tumor cells were plated and treated for 3 days with 10 µg/ml MAb 4D5 or 7C2 as described above. DNA was extracted, ³²P-end-labeled, and run on a 2% agarose gel containing 5µg/ml ethidium bromide. The gel was then dried and exposed to Kodak film. The formation of DNA ladders, a hallmark of apoptosis, was observed in BT474 breast tumor cells treated with 10µg/ml MAb 7C2 or MAb 4D5 for 3 days.

**Electron micrography studies.** BT474 cells were treated with 10µg/ml MAb 7C2 for 3 days, then fixed in 1.25% formaldehyde/1% glutaraldehyde in 0.1M cacodylate buffer. Post-fixation was performed in 2% osmium tetroxide in cacodylate buffer. The fixed cells were then end-block stained in uranyl acetate, dehydrated in graded concentrations of ethanol, and embedded in Eponet. Sections were cut utilizing a microtome and observed under a Philips CM12™ electron microscope. Highly shrunken cells displaying nuclear and cytoplasmic condensation, typical of apoptotic cells, were observed after treatment with MAb 7C2. The apoptotic cells eventually become phagocytosed by underlying cells.

The results of the experiments performed are shown in Figs. 5-14. Certain anti-ErbB2 MAbs induce apoptosis in human tumor cell lines which overexpress ErbB2 as evidenced by electron microscopy, annexin-V binding, cell cycle analysis of DNA content, DNA laddering and time-lapse videomicrography. Anti-ErbB2 MAbs 7C2 and 7F3, which recognize the same epitope on the ErbB2 extracellular domain, display the most potent pro-apoptotic effects. Anti-ErbB2 MAb 4D5, which recognizes a different ErbB2 epitope, induces a small amount of apoptosis in addition to its potent reduction in proliferation. Induction of apoptotic cell death by 7C2 or 4D5 appears to be independent of cell cycle. Inhibition of growth, either by serum deprivation or by treatment with 4D5, followed by treatment with 7C2 can result in complete cell death of the culture.

### EXAMPLE 3

### APOPTOSIS OF OVARIAN CELLS AND COMBINATION TREATMENT

**Methods.** SKOV3 ovarian cancer cells were seeded at a density 10⁶ per dish in 20x100 mm dishes and allowed to attach for 2-3 days. The medium was then removed and replaced with fresh medium alone or medium containing the appropriate anti-HER2 MAb. For studies on treatment with single MAb, cells were incubated with 10 µg/ml MAb 7C2 or 4D5 for 3 days. For antibody combination treatments, BT474 cells were treated first for 24 hr with 0.25 or 0.5 µg/ml MAb 7C2. Following this treatment, 10 µg/ml MAb 4D5 was added and the cells were incubated for 3 more days. Following each treatment, supernatants were individually collected and kept on ice, monolayers were detached by trypsinization and pooled with the corresponding supernatant. Cells were then centrifuged at 1200 rpm for 5 min at 4°C, the pellet resuspended in ice cold Ca²⁺ binding buffer (10 mM Hepes, pH 7.4, 140 mM NaCl, 2.5 mM CaCl₂) and aliquoted into 35 mm strainer-capped 12x75 tubes. Cells were stained with 1 µg/ml annexin V-FITC and 10 µg/ml propidium iodide (PI) and analyzed on a FACScan flow cytometer using FACSCONVERT CELLQUEST™ software (Becton Dickinson).

**Induction of apoptosis in ovarian cells.** The percent of remaining viable (annexin V negative/PI negative) cells after 3 days of treatment with MAb 7C2 was reduced by 48% (3.8 fold increase in annexin V binding) in the SKOV3 ovarian carcinoma line (Fig. 15).

**Combination treatment.** Induction of apoptosis by the sequential addition of MAbs 7C2 and 4D5 leads to additive effects compared to either antibody alone (Fig. 16). Treatment of BT474 breast tumor cells with 0.5 µg/ml MAb 7C2 followed by 10 µg/ml MAb 4D5 results in a reduction in viable (annexin V negative/PI negative) cells to 12%, compared to 68% or 29.1% with MAb 4D5 or 7C2 alone, respectively. This additive effect is also seen with a suboptimal dose of MAb 7C2, where treatment with 0.25 µg/ml 7C2: plus 10 µg/ml 4D5 leads to a decrease in viable cells to 37.5%, compared to 68% for MAb 4D5 alone and 77% for 7C2 alone. Simultaneous addition of MAbs 7C2 and 4D5 or addition of MAb 4D5 prior to 7C2 does not appear to lead to additive effects on cell death. Thus, without being bound by any one theory, the sequential application of MAb 7C2, then MAb 4D5, may be important for achieving an enhanced apoptotic effect.

### EXAMPLE 4

### IN VIVO EFFECTS OF ANTI-HER2 ANTIBODIES

A xenograft model of HER2 overexpressing human breast cancer was established to assess the effect of anti-HER2 MAbs in relation to HER2-overexpressing tumors. The model uses the BT474 cell line selected *in vivo* for growth in nude mice, BT474M1. Tumor bearing mice were treated twice weekly with monoclonal antibodies, 4D5, 7C2 or 7F3 or combinations of 4D5 with 7C2 or 7F3. Tumor growth was assessed by measuring tumor size twice weekly. The 4D5 monoclonal antibody had significant growth inhibitory effects on HER2 overexpressing xenograft tumors from the BT474M1 cell line. These results are similar to previously published results. The monoclonal antibodies 7C2 and 7F3 had modest growth inhibitory effects on their own, at the doses tested, and significantly enhanced the growth inhibitory effect of 4D5. None of the antibodies exhibited any toxic effect on the animals.

**Animal Model.** NCR.nu/nu mice (homozygous females, 4 weeks of age) were implanted subcutaneously with 0.72 mg sustained release 17βestradiol pellets to support the growth of tumor. Animals were inoculated by subcutaneous injection with 5 million BT474M1 tumor cells in MATRIGEL™ 24 hours after estrogen implantation. Animals were monitored daily for well being and tumors were measured twice weekly. Reports on tumor measurements were supplied as they were collected. Animals were weighed weekly to assess toxicity during the study. One hundred five (105) animals were inoculated and all animals were evaluated in the study.

**Treatment Protocol.** Animals were randomized to one of 7 treatment groups (15 animals per group). Treatment was initiated 6 days after inoculation of tumors. Tumor sizes for all animals and mean tumor sizes for each of the treatment groups were examined prior to beginning treatment to ensure consistency between groups. Treatment groups were:
1) Vehicle control injection - 100 µl by intraperitoneal (IP) injection twice weekly
2) Irrelevant antibody (αgp120) (isotype matched) - 10mg/kg in 100 μl IP twice weekly
3) MAb 7C2 - 10 mg/kg in 100 µl by IP injection twice weekly
4) MAb 7F3 - 10 mg/kg in 100 µl by IP injection twice weekly
5) MAb 4D5 - 10 mg/kg in 100 µl by IP injection twice weekly
6) MAb 7C2 (10mg/kg) + MAb 4D5 (10 mg/kg) - in 100 µl by IP injection weekly
7) MAb 7F3 (10 mg/kg) + MAb 4D5 (10 mg/kg) - in 100 µl by IP injection weekly

All treatment groups were treated twice weekly for a total of 10 treatments by intraperitoneal injection. Treatment groups 1-4 were euthanized at this point due to large tumor size. Treatment groups 5, 6 and 7 were continued and received a total of 16 antibody treatments. Throughout the study, animals were monitored daily for well being, twice weekly for tumor measurements and weekly for body weights. Individual animals data and mean data by treatment group was supplied as it became available.

**Termination of experiment.** After conclusion of treatment, animals in treatment groups 1-4 were euthanized due to some large tumor sizes within these treatment groups. Tumor volume was not allowed to exceed 4gms (4,000 mm³). No animals were observed with significant weight loss and weight loss greater than 15% loss of body weight from the weight at onset of treatment was never observed. At the conclusion of the experiment, all animals were euthanized.

**Results.** The monoclonal antibodies, 4D5, 7C2 and 7F3, directed against the HER2 growth factor receptor, were used in a mouse xenograft model which overexpresses the HER2 receptor. Antibodies were used alone and in combinations of growth inhibiting antibody (4D5) with apoptotic antibodies (7C2 and 7F3). The apoptotic antibodies (7C2 and 7F3) had a growth inhibitory effect early on in the study that was lost at later time points (Fig. 17). The growth inhibitory antibody, 4D5, had a marked growth inhibitory effect throughout the study, as has been reported in previous studies. The combination of 4D5 with either 7C2 or 7F3 potentiated the growth inhibitory effect significantly, with 4D5/7C2 being the best combination. There was one complete remission in the 4D5 alone treatment group and one complete remission in the 4D5/7C2 treatment group. The antibody control group (anti-gp120 MAb) was equivalent to the saline treated control group. Body weights initially increased after tumor inoculation, then were maintained through the remainder of the study. None of the antibodies exhibited any toxic effect on the animals.

## Claims

1. A composition for use in a method of therapeutic treatment in a mammal of a cancer **characterized by** overexpression of ErbB2, the method comprising administering to the mammal the composition comprising an effective amount of a first anti-ErbB2 antibody, which antibody binds to epitope 7C2/7F3, as defined in SEQ ID NO: 2, on Domain 1 of ErbB2 and induces cell death via apoptosis of a cancer cell of the mammal which overexpresses ErbB2 when the cancer cell is exposed to the antibody, the method further comprising administering to the mammal a second anti-ErbB2 antibody, which second antibody binds to epitope 4D5, as defined in SEQ ID NO: 4, on ErbB2 and inhibits growth of SKBR3 cells in cell culture by 50-100%, wherein the cancer cell of the mammal is exposed to the first antibody before it is exposed to the second antibody, by the method comprising sequential administration of the first antibody followed by the second antibody.

2. The composition for use according to claim 1, wherein the first antibody has complementarity determining regions (CDRs) of antibody 7C2, obtainable from deposit ATCC HB-12215.

3. The composition for use according to claim 1 or claim 2 wherein the second antibody has complementarity determining regions (CDRs) of antibody 4D5, obtainable from deposit ATCC CRL 10463.

4. The composition for use according to claim 1 wherein the cancer is breast cancer.

5. The composition for use according to claim 1, the method further comprising exposing the cell to a growth inhibitory agent.

6. The composition for use according to claim 1, the method further comprising exposing the cell to a chemotherapeutic agent.

7. The composition for use according to claim 1, the method further comprising exposing the cell to radiation.

8. The composition for use according to any one of the preceding claims wherein the mammal is human.

## Patentansprüche

1. Zusammensetzung zur Verwendung in einem Verfahren zur therapeutischen Behandlung von durch eine Überexpression von ErbB2 gekennzeichnetem Krebs in einem Säugetier, wobei das Verfahren das Verabreichen der Zusammensetzung, die eine wirksame Menge eines ersten Anti-ErbB2-Antikörpers umfasst, an das Säugetier umfasst, wobei der Antikörper an Epitop 7C2/7F3, wie in Seq.-ID Nr. 2 definiert, in Domäne 1 von ErbB2 bindet und mittels Apoptose einer ErbB2 überexprimierenden Krebszelle des Säugetiers Zelltod induziert, wenn die Krebszelle dem Antikörper ausgesetzt wird, wobei das Verfahren weiters das Verabreichen eines zweiten Anti-ErbB2-Antikörpers an das Säugetier umfasst, wobei der zweite Antikörper an Epitop 4D5, wie in Seq.-ID Nr. 4 definiert, auf ErbB2 bindet und das Wachstum von SKBR3-Zellen in Zellkultur um 50 bis 100 % hemmt, wobei die Krebszelle des Säugetiers gemäß dem Verfahren, das aufeinanderfolgende Verabreichung des ersten Antikörpers gefolgt von dem zweiten Antikörper umfasst, dem ersten Antikörper ausgesetzt wird, bevor sie dem zweiten Antikörper ausgesetzt wird.

2. Zusammensetzung zur Verwendung nach Anspruch 1, wobei der erste Antikörper komplementaritätsbestimmende Regionen (CDRs) von Antikörper 7C2 aufweist, der aus der Hinterlegung ATCC HB-12215 erhältlich ist.

3. Zusammensetzung zur Verwendung nach Anspruch 1 oder 2, wobei der zweite Antikörper komplementaritätsbestimmende Regionen (CDRs) von Antikörper 4D5 aufweist, der aus der Hinterlegung ATCC CRL 10463 erhältlich ist.

4. Zusammensetzung zur Verwendung nach Anspruch 1, wobei der Krebs Brustkrebs ist.

5. Zusammensetzung zur Verwendung nach Anspruch 1, wobei das Verfahren weiters das Aussetzen der Zelle gegenüber einem Wachstumshemmer umfasst.

6. Zusammensetzung zur Verwendung nach Anspruch 1, wobei das Verfahren weiters das Aussetzen der Zelle gegenüber einem Chemotherapeutikum umfasst.

7. Zusammensetzung zur Verwendung nach Anspruch 1, wobei das Verfahren weiters das Bestrahlen der Zelle umfasst.

8. Zusammensetzung zur Verwendung nach einem der vorangegangenen Ansprüche, wobei das Säugetier ein Mensch ist.

## Revendications

1. Composition pour une utilisation dans un procédé de traitement thérapeutique chez un mammifère d'un cancer **caractérisé par** la surexpression de ErbB2, le procédé comprenant l'administration au mammifère de la composition comprenant une quantité efficace d'un premier anticorps anti-ErbB2, anticorps qui se lie à l'épitope 7C2/7F3, tel que défini dans la SEQ ID N° 2, sur le domaine 1 de ErbB2 et induit la mort cellulaire par apoptose d'une cellule cancéreuse du mammifère qui surexprime ErbB2 lorsque la cellule cancéreuse est exposée à l'anticorps, le procédé comprenant en outre l'administration au mammifère d'un second anticorps anti-ErbB2, second anticorps qui se lie à l'épitope 4D5, tel que défini dans la SEQ ID N° 4, sur ErbB2 et inhibe la croissance de cellules SKBR3 en culture cellulaire de 50 à 100 %, la cellule cancéreuse du mammifère étant exposée au premier anticorps avant d'être exposée au second anticorps, par le procédé comprenant l'administration séquentielle du premier anticorps puis du second anticorps.

2. Composition pour une utilisation suivant la revendication 1, dans laquelle le premier anticorps a des régions de détermination de complémentarité (CDR) de l'anticorps 7C2, pouvant être obtenu auprès du dépôt ATCC HB-12215.

3. Composition pour une utilisation suivant la revendication 1 ou la revendication 2, dans laquelle le second anticorps a des régions de détermination de complémentarité (CDR) de l'anticorps 4D5, pouvant être obtenu auprès du dépôt ATCC CRL 10463.

4. Composition pour une utilisation suivant la revendication 1, le cancer étant le cancer du sein.

5. Composition pour une utilisation suivant la revendication 1, le procédé comprenant en outre l'exposition de la cellule à un agent inhibiteur de croissance.

6. Composition pour une utilisation suivant la revendication 1, le procédé comprenant en outre l'exposition de la cellule à un agent chimiothérapeutique.

7. Composition pour une utilisation suivant la revendication 1, le procédé comprenant en outre l'exposition de la cellule à un rayonnement.

8. Composition pour une utilisation suivant l'une quelconque des revendications précédentes, le mammifère étant un être humain.
